# EUROPEAN PATENT SPECIFICATION

(11) **EP 3 946 436 B1**
(45) Date of publication and mention of the grant of the patent: **30.04.2025**
(21) Application number: 20714678.8
(22) Date of filing: 26.03.2020
(51) Int. Cl.: A61K 39/00, A61P 35/00, C07K 16/00, C07K 16/28

(54) **PHOTOACTIVE ANTIBODIES**
PHOTOAKTIVE ANTIKÖRPER
ANTICORPS PHOTOACTIFS

(30) Priority: 26.03.2019 GB 201904188
(43) Date of publication of application: 09.02.2022
(73) Proprietor: UEA Enterprises Limited, Norwich NR4 7TJ (GB)
(72) Inventor: SACHDEVA, Amit, Norwich Norfolk NR4 7TJ (GB); BRIDGE, Thomas, Norwich Norfolk NR4 7TJ (GB)
(74) Representative: Appleyard Lees IP LLP
(86) International application number: PCT/GB2020/050815
(87) International publication number: WO 2020/193981

(56) References cited:
- WO-A1-2007/107764
- WO-A1-97/13782
- GUNNOO S.B. ET AL: "Creation of a gated antibody as a conditionally functional synthetic protein", NATURE COMMUNICATIONS, vol. 5, no. 1, 4388, 30 July 2014 (2014-07-30), XP055477110
- ARBELY E. ET AL.: "Photocontrol of tyrosine phosphorylation in mammalian cells via genetic encoding of photocaged tyrosine", J. AMER. CHEM. SOC., vol. 134, 2012, pages 11912 - 11915, XP002795936

## Description

### Field of the Invention

The invention relates to photoactivatable antibodies.

### Introduction

Chemists and biochemists have successfully designed molecular systems that can be controlled in a defined manner in response to external agents, such as pH, light and other small molecules (Nguyen, D. P.; Mahesh, M.; Elsasser, S. J.; Hancock, S. M.; Uttamapinant, C.; Chin, J. W., Genetic encoding of photocaged cysteine allows photoactivation of TEV protease in live mammalian cells. J. Am. Chem. Soc. 2014, 136 (6), 2240-3). Controlling the activity of small molecules and biomolecules has allowed development of molecular machines, novel drugs, and nano-delivery systems, which have found applications in medicine, biotechnology, and nanotechnology. Over the last two decades, monoclonal antibodies and antibody fragments have emerged as a major class of biotherapeutics. However, there are only a few studies where the control of binding of antibodies to their targets using an external agent has been reported (Gunnoo, S. B.; Finney, H. M.; Baker, T. S.; Lawson, A. D.; Anthony, D. C.; Davis, B. G., Creation of a gated antibody as a conditionally functional synthetic protein. Nat. Commun. 2014, 5, 4388).

Several antibody-based drugs are in use, or in clinical trials, for treatment of various diseases, particularly cancer. These antibodies identify and bind to cell surface receptors, directing cells towards apoptosis. The same cell surface receptors are often present on healthy cells, leading to severe side effects. If the binding of an antibody to its antigen could be triggered at the site of unhealthy cells, it would reduce the side effects of antibody-based therapeutics. One way of achieving this is using light.

Modulating the binding of antibody to its corresponding antigen using light presents an opportunity to gain spatial and temporal control over processes mediated by this highly diverse class of biomolecules, and would allow development of light-activated antibody-based molecular switches. Light-activated small molecule cytotoxic drugs are currently under investigation for treatment of cancer (Howerton, B. S.; Heidary, D. K.; Glazer, E. C., Strained ruthenium complexes are potent light-activated anticancer agents. J. Am. Chem. Soc. 2012, 134 (20), 8324-7.). Photodynamic therapy (PDT) is an emerging modality for the treatment of neoplastic and non-neoplastic diseases. Using photodynamic therapeutic approaches, photosensitizers are conjugated to biologically active molecules and are localized in target tissues, and subsequently activated with an appropriate wavelength of light. Light activation of the photosensitizers generates active molecular species, such as free radicals and singlet oxygen, which are toxic to target cells and tissues. Antibodies linked by chemical or peptide linkers to light activated small molecule drugs have also been developed (Mitsunaga, M.; Ogawa, M.; Kosaka, N.; Rosenblum, L. T.; Choyke, P. L.; Kobayashi, H., Cancer cell-selective in vivo near infrared photoimmunotherapy targeting specific membrane molecules. Nat. Med. 2011, 17 (12), 1685-91.).

The drawbacks with existing technologies are that these drugs are not selective for particular cells. Also, light sensitive conjugates can still exhibit cytotoxicity in the absence of light, due to binding of antibody to the cell surface antigen (Ochoa, M. C.; Minute, L.; Rodriguez, I.; Garasa, S.; Perez-Ruiz, E.; Inoges, S.; Melero, I.; Berraondo, P., Antibody-dependent cell cytotoxicity: immunotherapy strategies enhancing effector NK cells. Immunol. Cell Biol. 2017, 95 (4), 347-355).

Thus, there is a need for controlling the direct binding of antibody to its target and the invention is aimed at addressing this need. The invention is aimed at providing photoactive antibodies produced by site-specific incorporation of photocaged amino acids into the antigen binding site of the antibody. Such light-activated antibodies require light as well as specific cell surface receptors for their action.

The localised activation of such antibodies can help to minimise side effects. Documents helpful to understand the background of the invention are: WO2007/107764; WO97/13782; Gunnoo (2014), Nature Comm. 5(1):4388; and Arbely (2012), J. Am. Chem. Soc. 134:11912-11915.

### Summary

The invention is defined in the appended claims.

We provide an effective spatiotemporal control of the activity of antibodies, in particular therapeutic antibodies, by controlling the binding of the antibody to its target. This has been achieved by the inclusion of a modified amino acid in the sequence of the antibody at a position that is involved in the binding of the antibody to its target antigen. The modified amino acid includes a photoactive group, i.e. a photocaged group. The caging group hinders function of the antibody, that is binding of the antibody to its target antigen, until the molecule is liberated, or 'decaged', by exposure to light. Incorporation of the photocaged group into the antibody is achieved by genetic site-specific incorporation. Thus, the primary antibody sequence is modified by direct incorporation of the photocaged amino acid. Peptide or chemical linkers are not used to modify the antibody. In other words, the photocaged group is not conjugated to the antibody but it is integrated by site specific modification of the antibody sequence as explained herein.

In one aspect, we provide an antibody or fragment thereof wherein said antibody or fragment comprises a photocaged amino acid in one of the CDRs of its antigen binding region, wherein: i) the photocaged amino acid comprises a light-removable protection group; ii) the photocaged amino acid is present at a site in which the photocaged amino acid inhibits interaction and/or binding of the antibody to its antigen, wherein binding and/or interaction of the antibody to its antigen can be induced upon activation with a light source, and wherein the antibody or fragment thereof is conjugated to another moiety.

The photocaged amino acid is genetically encoded.

The amino acid may include a photoactive group selected from an o-nitrobenzyl functional group, or variants thereof.

The fragment may comprise a F(ab')2, Fab, Fv, sFv scFv dAbs.

The moiety may be selected from a half life extension moiety, label or toxic moiety.

The moiety may be conjugated to the antibody or fragment via an unnatural amino acid.

The antibody or fragment thereof may bind to a cell surface antigen.

The antibody or fragment thereof may bind to a tumor associated antigen.

The antibody or fragment thereof may bind to an immune checkpoint molecule.

We also provide a pharmaceutical composition comprising an antibody or fragment thereof as described above and optionally a pharmaceutically acceptable carrier.

We also provide an antibody or fragment thereof as described above or a pharmaceutical composition as described above for use in the treatment of disease, wherein: i) the antibody or fragment thereof is to be activated by irradiating a target tissue with a light source, optionally wherein the wavelength is 365nm; ii) the disease is selected from cancer, immune disease, neurological disease, inflammatory disease, allergy, transplant rejection, viral infection, immune deficiency, and other immune system-related disease cancer.

The cancer may be selected from bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, breast cancer, brain cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, kidney cancer, sarcoma of soft tissue, cancer of the urethra, cancer of the bladder, renal cancer, lung cancer, non-small cell lung cancer, thymoma, prostate cancer, mesothelioma, adrenocortical carcinoma, lymphomas, such as such as Hodgkin's disease, non-Hodgkin's, gastric cancer, leukemias such as ALL, CLL, AML, urothelial carcinoma leukemia and multiple myelomas. We also provide an in vitro method of reducing tumor cell growth and/or proliferation in a tumour cell or tissue comprising
a) contacting a tumor cell with an effective amount of an antibody or fragment thereof as described above or a pharmaceutical composition as described above, wherein the antibody targets an antigen present on the surface of a tumor cell and exerts an inhibitory effect on growth and/or proliferation of the tumor cell when bound to the antigen and/or wherein the antibody targets an antigen present on the surface of a tumor cell is internalised when bound to the antigen; and
b) irradiating the antibody or fragment with a light source to release the photocaging group and induce specific binding of the antibody to the target antigen; and
thereby inhibiting growth and/or proliferation of the tumor cell.

We also provide an in vitro method of killing a tumour cell, comprising:
a) contacting a tumor cell comprising a cell surface protein with a therapeutically effective amount of an antibody or fragment thereof as described above or a pharmaceutical composition as described above, wherein the antibody targets an antigen present on the surface of a tumor cell is internalised when bound to the antigen; and
b) irradiating the antibody or fragment with a light source to release the photocaging group and induce specific binding of the antibody to the target antigen wherein said antibody is internalised upon binding to the antigen; and
thereby killing the cell.

Irradiating the antibody or fragment comprises irradiating a tumor cellcomprises applying a suitable light source selected from the group consisting of a filtered conventional light source, a diode array and a laser.

We also disclose, but do not claim, a nucleic acid encoding an antibody or antibody fragment as described above.

We also provide a method for producing an antibody or antibody fragment as described above, comprising identifying an amino acid residue in one of the CDRS of the antigen binding region for site specific modification and introducing a photocaged group at said position, wherein i) the photocaged amino acid comprises a light-removable protection group; ii) the photocaged amino acid inhibits interaction and/or binding of the antibody to its antigen wherein binding and/or interaction of the antibody to its antigen can be induced upon activation with a light source.

We also provide a kit comprising the antibody or fragment thereof as described above, optionally comprising a LED wearable device or an LED implantable device.

### Brief Description of Figures

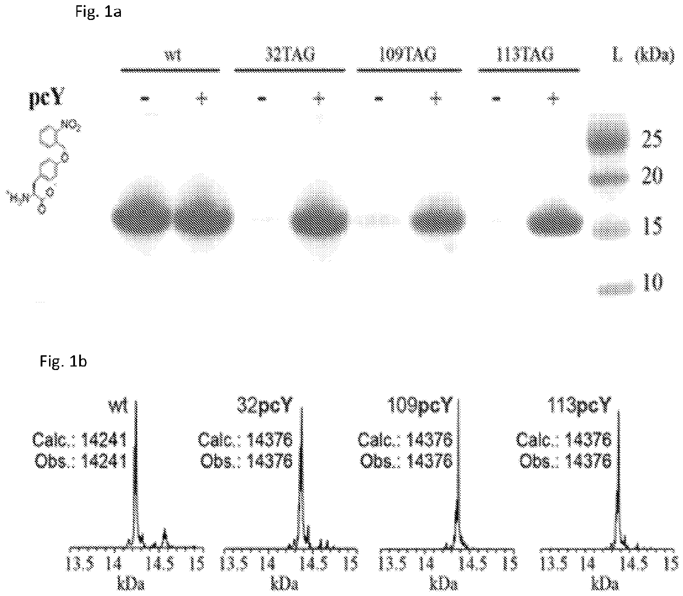

**Figure 1****.** Genetic site-specific incorporation of pcY in 7D12. **(A)** The expression of three amber mutants of 7D12, viz. 32TAG, 109TAG and 113TAG only occurs in the presence of pcY. Comparison of band intensities for amber mutants with wt7D12 shows efficient incorporation of pcY. **(B)** ESI-MS of amber mutants compared to wt7D12 confirms incorporation of pcY.

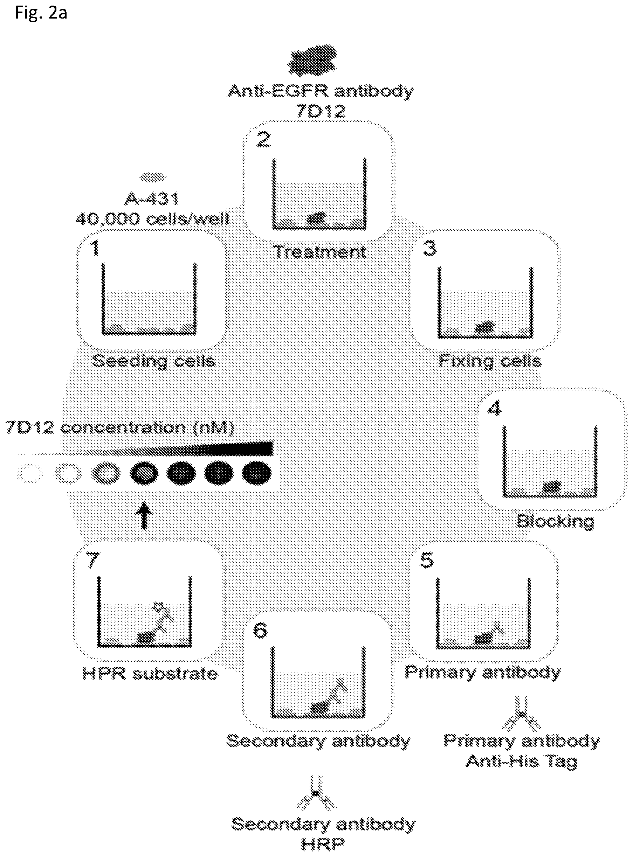

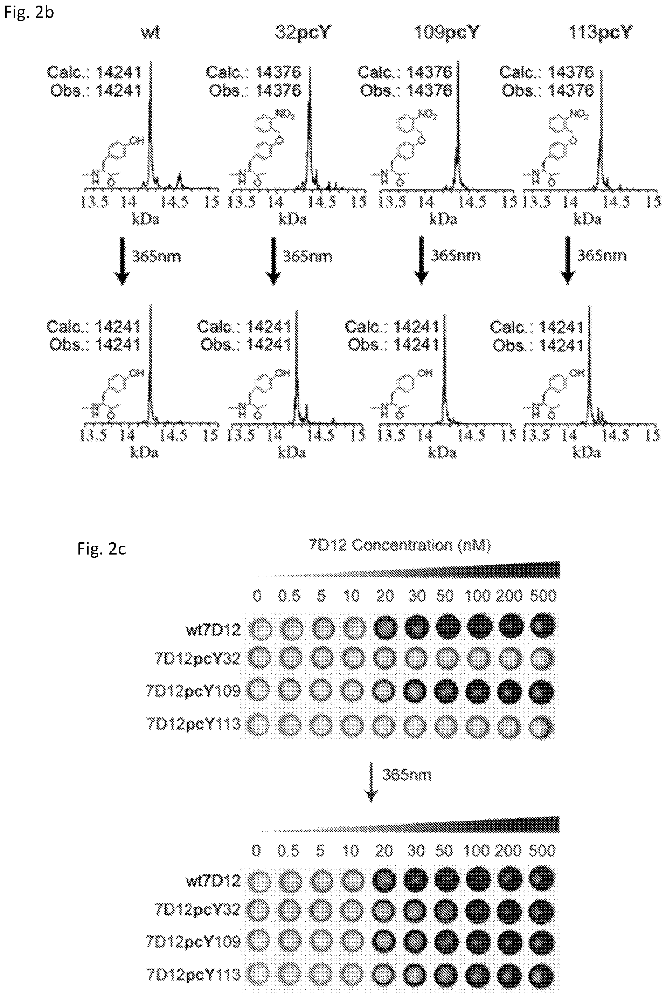

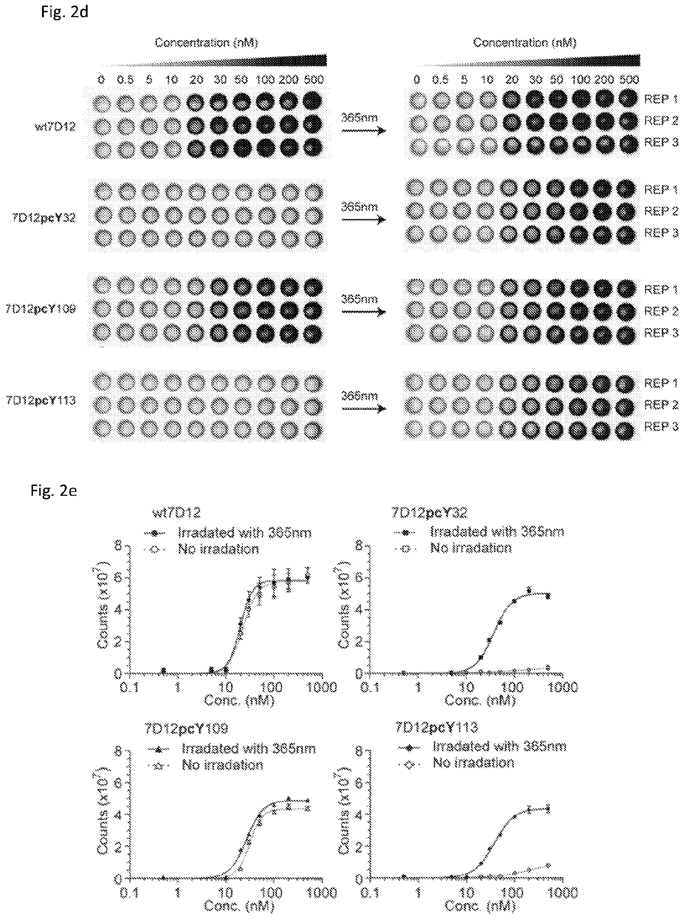

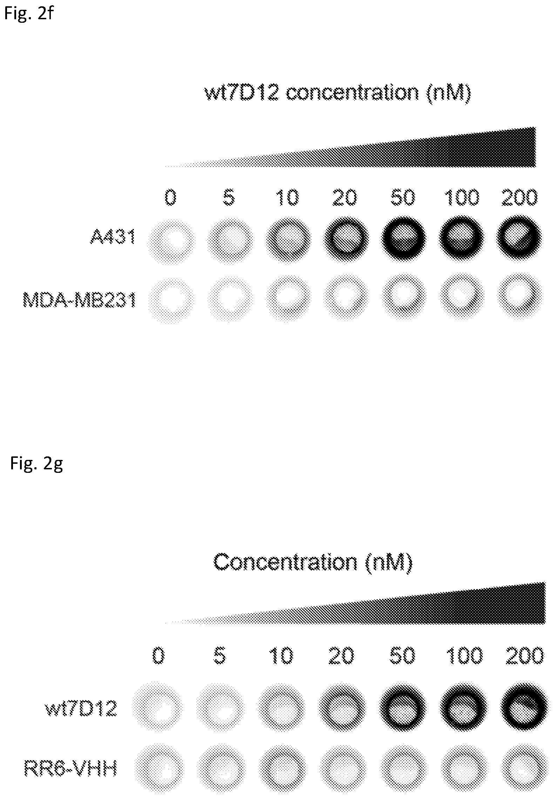

**Figure 2****.** Assessing the binding of photocaged mutants of His-tagged antibody to EGFR on the surface of cells. **(A)** Schematic representation of western blot on the surface of A431 cancer cells. 1. 40,000 cells were seeded into each well of a 96-well plate. 2. These cells were incubated with the complete media containing the antibody. 3. The antibody solution was replaced with 3.7% formaldehyde solution for fixing the cells. 4. This was followed by incubation with blocking solution. 5. Incubation with primary antibody specific for His6 tag. 6. Incubation with HRP-linked secondary antibody. 7. The substrate for HRP was added and the cells were imaged for chemiluminescence. **(B)** Comparison of ESI-MS of photocaged mutants of 7D12 before and after irradiation with 365 nm light confirms light-mediated decaging. **(C)** On-cell binding assay. The on-cell binding assay demonstrates that the presence of **pcY** at positions 32 and 113 in 7D12 inhibits its binding to EGFR. 7D12**pcY**109 mutant does not show inhibition in binding to EGFR. Binding of 7D12**pcY**32 and 7D12**pcY**113 is restored upon irradiation with 365 nm light. These experiments were performed in triplicate**(D)** On-cell binding experiments to assess the interaction of EGFR with mutants of 7D12 containing site-specifically incorporated **pcY** were performed in triplicates. On-cell assays performed on the surface of A431 cells demonstrates that the presence of **pcY** at positions 32 and 113 in 7D12 inhibits its binding to EGFR. However, 7D12**pcY**109 mutant shows binding affinity similar to wt7D12. The binding to 7D12**pcY**32 and 7D12**pcY**113 mutants is restored upon irradiation with 365 nm light. These experiments were performed in triplicates, REP1, REP2 and REP3, to ensure reproducibility of data.. **(E)** Chemiluminescence intensity was quantified using a CLARIOstar plate reader and plotted against concentration of 7D12, where X-axis is in log scale; the data was fitted to a sigmoidal nonlinear curve using GraphPad. **(F)** Western blot on the cell surface assesses specific binding between His6-tagged antibody fragment and cell surface receptor. Chemiluminescence signal observed when wt7D12 was incubated with an MDA-MB231, a cell line with low levels of EGFR, was significantly lower compared to the signal from A431 cancer cell line, that has higher levels of EGFR. **(G)** His6-tagged antibody fragments do not bind non-specifically to the surface of A431 cells. Near background level chemiluminescence signal was observed when an unrelated His6-tagged antibody fragment, RR6-VHH, is incubated with A431 cancer cells.

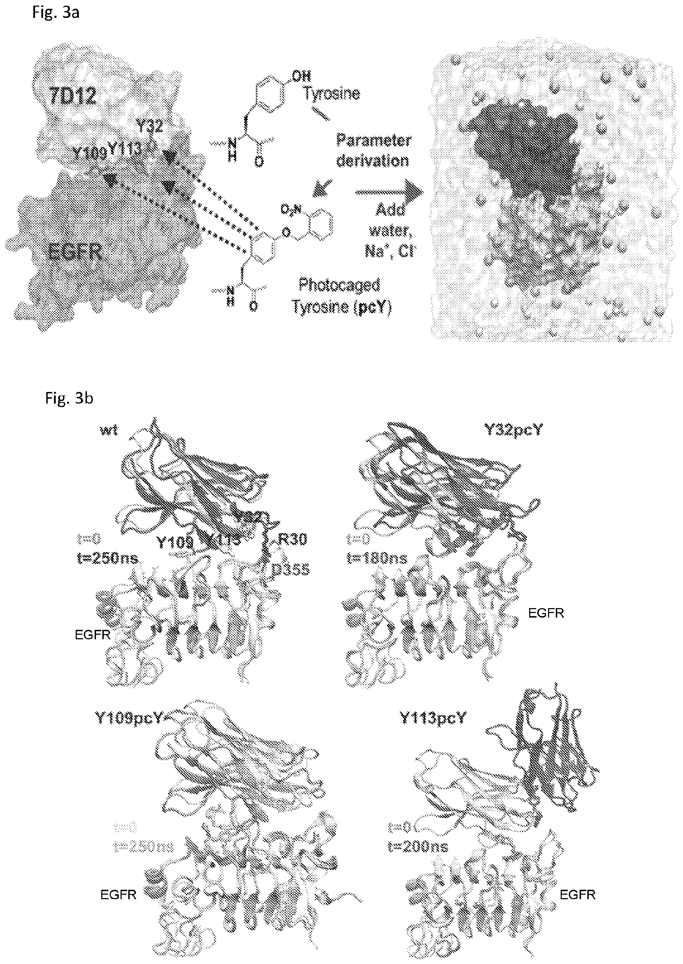

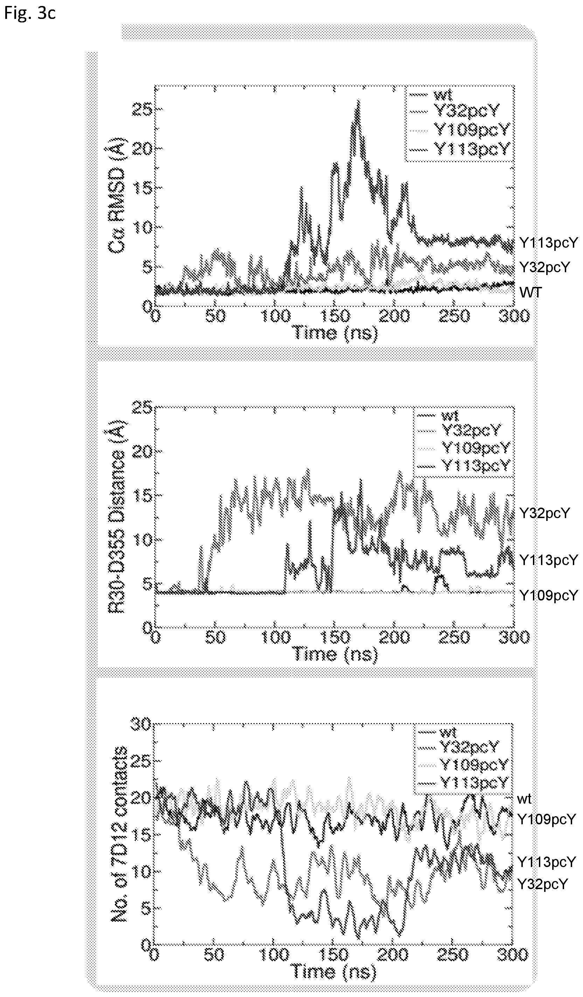

**Figure 3****.** MD simulations of wt7D12 and three amber mutants (7D12pcY32, 7D12pcY109, and 7D12pcY113) show that wt7D12 and 7D12pcY109 form more stable complexes with EGFR domain III as compared to 7D12pcY32 and 7D12pcY113. **(A)** The crystal structure for 7D12-EGFR domain III complex (PDB ID: 4KRL) was used as the starting structure for wt7D12-EGFR domain III simulations, as well as to generate the three mutants, by modelling in pcY at positions 32, 109, and 113 respectively. The complexes were simulated in the presence of explicit water and ions.
   **(B)** Snapshots taken during the simulation for each system, (EGFR- yellow for all, wt7D12 - black, 7D12pcY32 - red, 7D12pcY109 - green, 7D12pcY113 - blue) superimposed on the starting structure (light colours) highlight the extent of motion of 7D12.
   **(C)** Top panel: Root mean square deviations (RMSDs) from starting structure for protein Cα atoms during 300 ns MD simulations for each of the four systems. Middle panel: The R30-D355 salt-bridge (residues shown in snapshot of wt7D12 in B), monitored as the distance between the R30 guanidine C and the D355 carboxyl C, breaks frequently in the 7D12pcY32 and 7D12pcY113 systems. The number of 7D12-EGFR domain III contacts during 300ns of simulations, measured as the number of 7D12 heavy atoms within 3.5 Å of EGFR heavy atoms, show that 7D12pcY32 and 7D12pcY113 form lesser contacts with EGFR for long periods, compared to the complexes with wt7D12 and 7D12pcY109. These observations suggest that the presence of pcY at positions 32 and 113 destabilizes the 7D12-EGFR domain III complex.

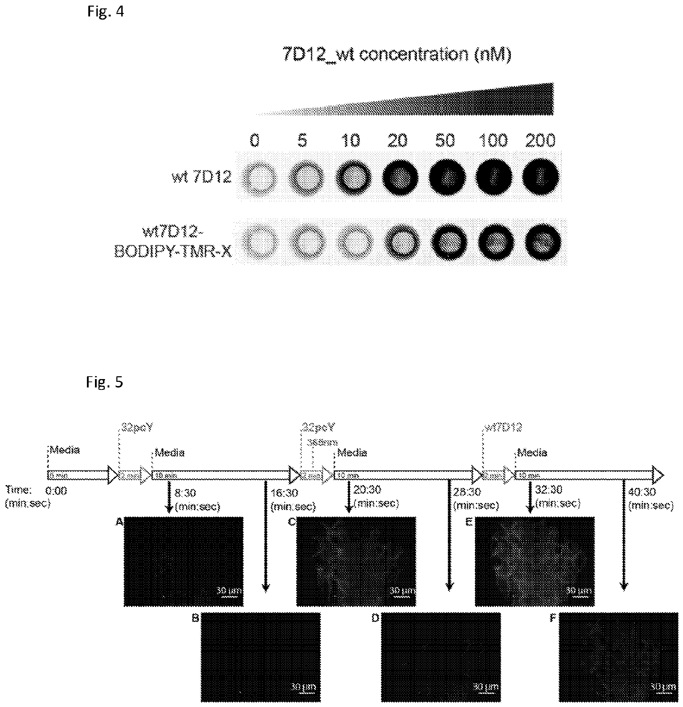

**Figure 4****.** Comparison of binding of labelled and unlabelled wt7D12 to EGFR assessed using western blot on the surface of A431 cells. Binding of wt7D12 is reduced by ~1.5-fold due to the presence of the BODIPY-TMR-X label.
**Figure 5****.** Light-mediated delivery of fluorophores by photoactive antibodies on live A431 cells. **(A)** Labelled 7D12pcY32 is injected at 5 min. Near-background fluorescence is observed 1.5 min after passing labelled 7D12pcY32 over live A431 cells demonstrating that due to the presence of caged group 7D12pcY32 does not bind to the cell surface. **(B)** Background fluorescence before re-injecting labelled 7D12pcY32. **(C)** Labelled 7D12pcY32 was injected at 17 min and the irradiated with 365 nm light at 18 min. Significant fluorescence was observed 1.5 min after stopping the injection of labelled 7D12pcY32, demonstrating light-dependent localization of 7D12 on the surface of A431 cells. **(D)** Fluorescence from 7D12 reduces to background level presumably due to endocytosis of 7D12 and degradation of the fluorophore. **(E)** Labelled wt7D12 was injected at 29 min. Significant fluorescence observed 1.5 min after stopping the injection of labelled wt7D12 due to localization of labelled wt7D12 on the surface of A431 cells. **(F)** Fluorescence from wt7D12 slowly reduces to near background level presumably due to endocytosis of 7D12 and degradation of the fluorophore.

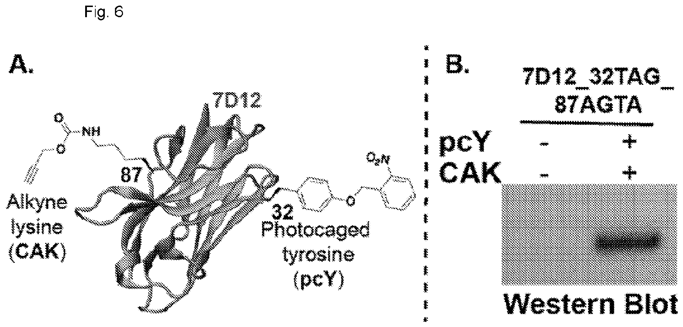

**Figure 6****.** Homogenously labelled photoactive antibody-drug conjugates (ADCs). Site-specific incorporation of two distinct unnatural amino acids into antibody fragment, 7D12. **(A)** Chemical structures of photocaged tyrosine (pcY) and alkyne lysine (CAK) incorporated at positions 32 and 87 in 7D12. **(B)** Western blot demonstrates that incorporation of pcY and CAK at positions 32 and 87, respectively are dependent on addition of these unnatural amino acids.

### Detailed Description

The technical disclosure set out below may in some respects go beyond the scope of the claims. Elements of the disclosure which do not fall within the scope of the claims are provided for information.

The present disclosure will now be further described. In the following passages, different aspects of the disclosure are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Generally, nomenclatures used in connection with, and techniques of, cell and tissue culture, pathology, oncology, molecular biology, immunology, microbiology, genetics and protein and nucleic acid chemistry and hybridization described herein are those well-known and commonly used in the art. The methods and techniques of the present disclosure are generally performed according to conventional methods well-known in the art and as described in various general and more specific references that are cited and discussed throughout the present specification unless otherwise indicated. See, e.g., Green and Sambrook et al., Molecular Cloning: A Laboratory Manual, 4th ed., Cold Spring Harbor Laboratory Press, Cold Spring Harbor, N.Y. (2012); Therapeutic Monoclonal Antibodies: From Bench to Clinic, Zhiqiang An (Editor), Wiley, (2009); and Antibody Engineering, 2nd Ed., Vols. 1 and 2, Ontermann and Duebel, eds., Springer-Verlag, Heidelberg (2010).

Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients.

In one aspect, we provide an antibody or fragment thereof wherein said antibody or fragment comprises a photocaged amino acid in its antigen binding region.

The antibody is thus modified by site specific modification to include an amino acid its sequence which is linked to a photocaging group. The photocaged group is not conjugated to the native amino acid sequence by using a chemical or peptide linker. Instead, the photocaged amino acid is genetically encoded and therefore forms part of the resulting amino acid sequence.

Genetic code expansion has enabled site-specific incorporation of several unnatural amino acids, including amino acids containing bio-orthogonal functional groups, photo-reactive amino acids and photocaged amino acids, into proteins (Chin, J. W., Expanding and reprogramming the genetic code of cells and animals. Annu. Rev. Biochem. 2014, 83, 379-408) and (Wang, L.; Xie, J.; Schultz, P. G., Expanding the genetic code. Annu. Rev. Biophys. Biomol. Struct. 2006, 35, 225-249). Photocaged unnatural amino acids, in particular, have been employed to control the activity of several biomolecules including transcription factors, kinases, proteases and inteins. Methods to encode unnatural amino acids can use orthogonal amino acyl tRNA synthetase/tRNA pairs and orthogonal quadruplet decoding ribosomes. In the orthogonal tRNA synthetase/tRNA approach the unnatural amino acid is detected by the altered specificity of the orthogonal synthetase and used to amino acylate the orthogonal tRNA. During translation the orthogonal tRNA will then introduce the unnatural amino acid in response to a unique or "non-sense" codon. A non-sense codon may be an amber stop codon (UAG), an opal stop codon (UGA) or an ochre stop codon (UAA) or quadruplet codons such as AGUA, AGGA, UAGA. Multiple orthogonal tRNA synthetase/tRNA pairs have been developed including but not limited to; tyrosyl-tRNA synthetase (TyrRS)/tRNA^{Tyr} from *Methanocaldococcus jannaschii,* lysine-RS/tRNA^{Lys} pair from *Pyrococcus horikoshii,* a glutamine-RS/tRNA^{Glu} pair from *Methanosarcina mazei,* pyrrolysine-RS/tRNA^{Pyl} pair from *Methanosarcina species,* a heterologous pair consisting of a leucyl-tRNA synthetase from *Methanobacterium thermoautotrophicum* and a mutant tRNA^{Leu} derived from *Halobacterium sp.,* and pair consisting of P. *horikoshii* proline-RS and three engineered suppressors tRNA^{Pro} from *Archaeoglobus fulgidu.*

Here, we genetically encode photocaged tyrosine at specific locations in an exemplary antibody fragment, 7D12. The identity of the modified proteins was confirmed by mass spectrometry. We have shown that an unnatural amino acid, i.e. a photocaged amino acid, can be genetically incorporated into an antibody molecule at a specific location and that this in turn can prevent binding of the antibody to its target antigen. Binding of the antibody to its target antigen is activated by irradiation with light.

Photoremovable protecting groups, or photocages, are groups that are covalently linked to a target molecule to inhibit its activity. The addition of 'caging' group in a key position in the antibody sequence important for antigen binding, can achieve effective spatiotemporal control of activity of the antibody molecule. The caging group hinders binding of the antibody to its target until the molecule is liberated, or 'decaged', by exposure to a specific wavelength of light.

Thus, the antibody of interest is rendered biologically inactive (caged) by the chemical modification with a protecting group within its sequence as it can no longer bind to its target antigen. The chemical modification can be removed (decaged) with light by irradiation of a suitable wavelength. This leads to the release of the caging group from the antibody, and the removal of the photocaging group results in binding of the antibody to its target.

Using the genetic incorporation approach the unnatural amino acid can be incorporated at any site within the antibody sequence. Routine mutagenesis techniques can be used to incorporate a nonsense codon which is then recognised by the orthogonal tRNA synthetase/tRNA pair and the unnatural amino acid is introduced. The photocaged amino acid may be introduced at a site in which it inhibits interaction and/or binding of the antibody to its antigen. The photocaged amino acid is introduced in one of the CDRs of the antibody. The skilled person would be able to identify suitable sites for inhibiting interaction and/or binding using routine techniques including analysis of X-ray crystallographic structures of the antibody and antigen interaction, molecular dynamic simulations of the antibody and antigen interaction, or a combination of these techniques.

It is also possible to incorporate one or more unnatural amino acids into the antibody sequence. This may comprise one or more of the same unnatural amino acids or one or more of different unnatural amino acids. By using multiple orthogonal tRNA synthetase/tRNA pairs it is possible to incorporate different unnatural amino acids into the antibody sequence. In an embodiment the antibody comprises one or more photocaged amino acids in the antigen binding site. In an embodiment, the antibody comprises one or more photocaged amino acids in the antigen binding site and one or more further unnatural amino acids. The further unnatural amino acid/s may comprise a bio-orthongonal functional group which can be used to attach a further molecule to the antibody, this may be particularly useful for antibody drug conjugates (ADCs). Functional groups such as an azide group, an alkyne group, a tetrazine group and or a strained alkene group. A strained alkene group may be for example a cyclopropane group. The unnatural amino acid/s may comprise a functional group such as a thiol group, and/or a carbonyl group which may be used to attach a further molecule to the antibody.

Photocaged antibodies, whose binding is inhibited by light-removable protection groups, thus represent an important molecular switch for antibody activity which can be exploited in therapeutic applications as disclosed herein.

In one embodiment, the functional photocaging group is selected from an o-nitrobenzyl group, or variants thereof. The photocaging group may be selected from a nitrophenethyl group, a nitroveratryl group, an arylcarbonylmethyl group, a phenylacyl group, an o-alkylphenacyl group, a p-hydroxyphenacyl group, a nitrobenzyl group, a coumarin-4-ylmethyl group. The functional group may be light responsive and selected from terazine, azobenzene, diazirine or benzophenone. In another embodiment, the group is selected from tert-butoxycarbonyl (Boc), alkyne or azide. In one embodiment, the group is an o-nitrobenzyl group.

The inventors have shown that modification of a single residue in this way leads to an antibody molecule whose binding to the antigen can be controlled by light. As the modification affects only a single residue, the modified antibody only differs by about 0.1 kDa in molecular weight from the parent i.e. non-modified antibody.

The term "antibody" as used herein expressly includes antibody fragments. The term "antibody" broadly refers to any immunoglobulin (Ig) molecule, or antigen binding portion thereof, comprised of four polypeptide chains, two heavy (H) chains and two light (L) chains, or any functional fragment, mutant, variant, or derivation thereof, which retains the essential epitope binding features of an Ig molecule. Such mutant, variant, or derivative antibody formats are known in the art.

In a full-length antibody, each heavy chain is comprised of a heavy chain variable region or domain (abbreviated herein as HCVR) and a heavy chain constant region. The heavy chain constant region is comprised of three domains, CH1, CH2 and CH3. Each light chain is comprised of a light chain variable region or domain (abbreviated herein as LCVR) and a light chain constant region. The light chain constant region is comprised of one domain, CL.

The heavy chain and light chain variable regions can be further subdivided into regions of hypervariability, termed complementarity determining regions (CDR), interspersed with regions that are more conserved, termed framework regions (FR). Each heavy chain and light chain variable region is composed of three CDRs and four FRs, arranged from amino-terminus to carboxy-terminus in the following order: FR1, CDR1, FR2, CDR2, FR3, CDR3, FR4. Immunoglobulin molecules can be of any type (e.g., IgG, IgE, IgM, IgD, IgA and IgY), class (e.g., IgG 1, IgG2, IgG 3, IgG4, IgA1 and IgA2) or subclass.

The term "CDR" refers to the complementarity-determining region within antibody variable sequences. There are three CDRs in each of the variable regions of the heavy chain and the light chain, which are designated CDR1, CDR2 and CDR3, for each of the variable regions. The term "CDR set" refers to a group of three CDRs that occur in a single variable region capable of binding the antigen. The exact boundaries of these CDRs can be defined differently according to different systems known in the art.

The Kabat Complementarity Determining Regions (CDRs) are based on sequence variability and are the most commonly used (Kabat et al., (1971) Ann. NY Acad. Sci. 190:382-391 and Kabat, et al., (1991) Sequences of Proteins of Immunological Interest, Fifth Edition, U.S. Department of Health and Human Services, NIH Publication No. 91-3242). Chothia refers instead to the location of the structural loops (Chothia and Lesk J. Mol. Biol. 196:901 -917 (1987)). The Kabat numbering system is generally used when referring to a residue in the variable domain (approximately residues 1-107 of the light chain and residues 1 -113 of the heavy chain).

The system described by Kabat is used herein unless otherwise specified. The terms "Kabat numbering", "Kabat definitions" and "Kabat labeling" are used interchangeably herein. These terms, which are recognized in the art, refer to a system of numbering amino acid residues which are more variable (i.e., hypervariable) than other amino acid residues in the heavy and light chain variable regions of an antibody, or an antigen binding portion.

A chimeric antibody is a recombinant protein that contains the variable domains including the complementarity determining regions (CDRs) of an antibody derived from one species, preferably a rodent antibody, while the constant domains of the antibody molecule are derived from those of a human antibody. For veterinary applications, the constant domains of the chimeric antibody may be derived from that of other species, such as a cat or dog.

A humanized antibody is a recombinant protein in which the CDRs from an antibody from one species; e.g., a rodent antibody, are transferred from the heavy and light variable chains of the rodent antibody into human heavy and light variable domains (e.g., framework region sequences). The constant domains of the antibody molecule are derived from those of a human antibody. In certain embodiments, a limited number of framework region amino acid residues from the parent (rodent) antibody may be substituted into the human antibody framework region sequences.

The term "antigen binding site" refers to the part of the antibody or antibody fragment that comprises the area that specifically binds to an antigen. An antigen binding site may be provided by one or more antibody variable domains. Preferably, an antigen binding site is comprised within the associated VH and VL of an antibody or antibody fragment.

A antibody or fragment thereof, "which binds" or is "capable of binding" an antigen of interest, , is one that binds the antigen with sufficient affinity such that the antibody is useful as a therapeutic agent in targeting a cell or tissue expressing the antigen.

The term "specific binding" or "specifically binds to" or is "specific for" a particular polypeptide or an epitope on a particular polypeptide target as used herein can be exhibited, for example, by a molecule having a KD for the target of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater. In one embodiment, the term "specific binding" refers to binding where a molecule binds to a particular polypeptide or epitope on a particular polypeptide without substantially binding to any other polypeptide or polypeptide epitope.

The term "epitope" or "antigenic determinant" refers to a site on the surface of an antigen (to which an immunoglobulin, antibody or antibody fragment, specifically binds. Generally, an antigen has several or many different epitopes and reacts with many different antibodies. The term specifically includes linear epitopes and conformational epitopes. Epitopes within protein antigens can be formed both from contiguous amino acids (usually a linear epitope) or non-contiguous amino acids juxtaposed by tertiary folding of the protein (usually a conformational epitope). Epitopes formed from contiguous amino acids are typically, but not always, retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods for determining what epitopes are bound by a given antibody or antibody fragment (i.e., epitope mapping) are well known in the art and include, for example, immunoblotting and immunoprecipitation assays, wherein overlapping or contiguous peptides from are tested for reactivity with a given antibody or antibody fragment. An antibody binds "essentially the same epitope" as a reference antibody, when the two antibodies recognize identical or sterically overlapping epitopes. The most widely used and rapid methods for determining whether two epitopes bind to identical or sterically overlapping epitopes are competition assays, which can be configured in different formats, using either labelled antigen or labelled antibody.

An antibody fragment is a portion of an antibody, for example as F(ab')2, Fab, Fv, sFv and the like. Functional fragments of a full length antibody retain the target specificity of a full length antibody. Recombinant functional antibody fragments, such as Fab (Fragment, antibody), scFv (single chain variable chain fragments) and single domain antibodies (dAbs) have therefore been used to develop therapeutics as an alternative to therapeutics based on mAbs.

scFv fragments (~25kDa) consist of the two variable domains, VH and VL. Naturally, VH and VL domain are non-covalently associated via hydrophobic interaction and tend to dissociate. However, stable fragments can be engineered by linking the domains with a hydrophilic flexible linker to create a single chain Fv (scFv).

The smallest antigen binding fragment is the single variable fragment, namely the VH or VL domain. Binding to a light chain/heavy chain partner respectively is not required for target binding. Such fragments are termed single domain antibodies. A single domain antibody (~12 to 15 kDa) therefore has either the VH or VL domain and does not comprise other parts of an antibody. VH and VL domains respectively are capable of binding to an antigen. The antigen-binding entity of an antibody, reduced in size to one single domain (corresponding to the VH or VL domain), is generally referred to as a "single-domain antibody" or "immunoglobulin single variable domain". Single domain antibodies derived from camelid heavy chain only antibodies that are naturally devoid of light chains as well as single domain antibodies that have a human heavy chain domain have been described (Muyldermans J Biotechnol. 2001 Jun; 74(4): 277-302; Holliger Nat Biotechnol. 2005 Sep; 23(9):1126-36).

Antigen binding single VH domains have also been identified from, for example, a library of murine VH genes amplified from genomic DNA from the spleens of immunized mice and expressed in E. coli (Ward et al., 1989, Nature 341: 544-546). Ward et al. named the isolated single VH domains "dAbs," for "domain antibodies." The term "dAb" generally refers to a single immunoglobulin variable domain (VH, VHH or VL) polypeptide that specifically binds antigen. The terms "single domain antibody (sdAb), variable single domain or immunoglobulin single variable domain (ISV)" are thus all well known in the art and describe the single variable fragment of an antibody that binds to a target antigen.

Thus, the various aspects described herein relate to a full length antibody, such as a monoclonal antibody, and also relate to an antibody fragment which may be selected from a F(ab')2, Fab, Fv, sFv, scFv, heavy chain, sdAb or any peptide derived from a full length antibody that retains specific binding affinity to its antigen.

The term "isolated" protein or polypeptide refers to a protein or polypeptide that is substantially free of other proteins or polypeptides, having different antigenic specificities. Moreover, protein or polypeptide may be substantially free of other cellular material and/or chemicals. Thus, the protein, nucleic acids and polypeptides described herein are preferably isolated. "Isolated nucleic acid molecule" means a DNA or RNA of genomic, mRNA, cDNA, or synthetic origin or some combination thereof which is not associated with all or a portion of a polynucleotide in which the isolated polynucleotide is found in nature, or is linked to a polynucleotide to which it is not linked in nature.

In one embodiment, the antibody or antibody fragment includes a single modification in its amino acid sequence, that is it includes only one amino acid that has a photocaged group. In another embodiment, the antibody or antibody fragment includes two or more amino acids that have a photocaged group. The modification is located in the antigen binding site of the antibody. In other words, the residue selected contributes to or mediates antigen binding. The residue is located in CDR1, CDR2 or CDR3. Disclosed herein but not forming part of the inveniton, the residue is not located in a CDR region, but mediates binding or contributes to specific binding to the antigen. Residues can be identified using structural analysis as described in the examples. Such methods are known in the art.

Thus, according to the embodiments described herein, a residue that interacts with the binding to the target antigen is modified and this modification, by genetic introduction of an amino acid with a photocaged group, prevents binding of the antibody to its target antigen or reduces binding of the antibody to the target antigen. Expose to light of a suitable wavelength, depending on the photocaged group used, results in decaging of the photocaged residue and the antibody binds to its target.

The antibody or fragment is conjugated to another moiety. Said moiety is selected from a half life extension moiety, label or therapeutic moiety, such as a drug, an enzyme or a toxin. In one embodiment, the therapeutic moiety is a toxin, for example a cytotoxic radionuclide, chemical toxin or protein toxin. The label may any label to aid in detection or isolation/purification of the antibody, such as radioisotopes, enzymatic proteins, fluorophores and fluorescent dyes.

The moiety can be linked to the heterodimeric protein, e.g. antibody, using linkers known in the art, e.g. via a chemical or peptide linker. The linkage can be covalent or non-covalent. An exemplary covalent linkage is via a peptide bond. In some embodiments, the linker is a polypeptide linker (L). Suitable linkers include for example a linker with GS residues such as (Gly4Ser)n. Other linkage/conjugation techniques include cysteine conjugation, e.g. for cysteine-based site specific antibody conjugation to a toxic payload.

In one embodiment, the moiety may be conjugated to the antibody or fragment via an unnatural amino acid. The unnatural amino acid may be site specifically genetically encoded. It may comprise a functional group such as an azide group, an alkyne group, a tetrazine group and/or a strained alkene. The unnatural amino acid may be selected from one or more of *p*-acetylphenylalanine, alkyne lysine, *p*-azidomethyl-_{L}-phenylalanine, *p*-azido-_{L}-phenylalanine *N*6-((2-azidoethoxy)carbonyl)-_{L}-lysine.The skilled person would be able to determine a suitable site for incorporation of the unnatural amino acid depending on the moiety to be attached.

The antibody or fragment may be modified to increase half-life, for example by a chemical modification, especially by PEGylation, or by incorporation in a liposome or using a serum albumin protein.

The antibody or fragment may be a therapeutic antibody or a diagnostic antibody (i.e. an antibody that does not have a therapeutic effect, but can be used for diagnostic purposes).

In one embodiment, the antibody or fragment binds to a cell surface antigen.

In one embodiment, the antibody or fragment binds to a tumor associated antigen (TAA). Tumor antigens can be loosely categorized as oncofetal (typically only expressed in fetal tissues and in cancerous somatic cells), oncoviral (encoded by tumorigenic transforming viruses), overexpressed/ accumulated (expressed by both normal and neoplastic tissue, with the level of expression highly elevated in neoplasia), cancer-testis (expressed only by cancer cells and adult reproductive tissues such as testis and placenta), lineage-restricted (expressed largely by a single cancer histotype), mutated (only expressed by cancer as a result of genetic mutation or alteration in transcription), posttranslationally altered (tumor-associated alterations in glycosylation, etc.), or idiotypic (highly polymorphic genes where a tumor cell expresses a specific "clonotype", i.e., as in B cell, T cell lymphoma/leukemia resulting from clonal aberrancies).

In one embodiment, the tumor associated antigen is selected from PSMA, Her2, Her3, CD123, CD19, CD20, CD22, CD23, CD74, BCMA, CD30, CD33, CD52, EGRF, CECAM6, CAXII, CD24, ETA, MAGE, Mesothelin, cMet, TAG72, MUC1, MUC16, STEAP, Ephvlll, FAP, GD2, IL-13Ra2, L1-CAM, PSCA, GPC3, gpA33, CA-125, gangliosides G(D2), G(M2) and G(D3), Ep-CAM, CEA, bombesin-like peptides, PSA, HER2/neu, epidermal growth factor receptor (EGFR), erbB2, erbB3, erbB4, CD44v6, Ki-67, cancer-associated mucin, VEGF, VEGFRs (e.g., VEGFR3), estrogen receptors, Lewis-Y antigen, TGFβ1, IGF-1 receptor, EGFα, c-Kit receptor, transferrin receptor, IL-2R, TAG-72 and CO17-1A.

In one embodiment, the antibody or fragment binds to EGFR. In one embodiment, the fragment is a VH or VHH and binds to EGFR. In one embodiment, the fragment is 7D12. In one embodiment, 7D12 includes a modified tyrosine. In one embodiment, 7D12 includes a modified tyrosine at positions 32 and/or 113.

In yet another embodiment, the antibody is an antibody that inhibits tumor cell growth and/or proliferation through binding to its antigen. For example, such an antibody may be selected from IMC-C225, EMD 72000, OvaRex Mab B43.13, anti-ganglioside G(D2) antibody ch14.18, CO17-1A, trastuzumab^{®}, cetuximab^{®}, rhuMAb VEGF, sc-321, AF349, BAF349, AF743, BAF743, MAB743, AB1875, Anti-Flt-4AB3127, FLT41-A, rituximab, 2C3, CAMPATH 1H, 2G7, Alpha IR-3, ABX-EGF, MDX-447, anti-p75 IL-2R, anti-p64 IL-2R, and 2A11.

In one embodiment, the antibody or fragment is an inhibitor of an immune checkpoint molecule. This may be selected from an inhibitor of one or more of PD-1, PD-L1, PD-L2, CTLA-4, TIM-3, CEACAM, VISTA, BTLA, TIGIT, LAIR1, CD160, 2B4 or TGFR beta. In another embodiment, the antibody may be an activator of a costimulatory molecule selected, for example, from an agonist of one or more of OX40, OX40L, CD2, CD27, CDS, ICAM-1, LFA-1 (CD11a/CD18), ICOS (CD278), 4-1BB (CD137), GITR, CD30, CD40, BAFFR, HVEM, CD7, LIGHT, NKG2C, SLAMF7, NKp80, CD160, B7-H3 or CD83 ligand, CD3, CD8, CD28, CD4 or ICAM-1.

The antibody may target tumor cell(s). A tumor cell comprises one or more cancer cells, or a mass of cancer cells, and can also encompass cells that support the growth and/or propagation of a cancer cell, such as vasculature and/or stroma, but not necessarily macrophages. For instance, therefore, the present invention envisages compositions and methods for reducing growth and/or proliferation of a tumor cell in a subject, wherein tumoricidial antibodies bind with specificity to cell surface epitopes (or epitopes of receptor-binding molecules) of a cancer cell or a cell that is involved in the growth and/or propagation of a cancer cell such as a cell within the vasculature of a tumor or blood vessels that supply tumors and/or stromal cells.

In another aspect, the disclosure relates to a pharmaceutical composition comprising an antibody or fragment thereof described herein and optionally a pharmaceutically acceptable carrier.

The pharmaceutically acceptable carrier or vehicle can be particulate, so that the compositions are, for example, in tablet or powder form. The term "carrier" refers to a diluent, adjuvant or excipient, with which a drug antibody conjugate of the present invention is administered. Such pharmaceutical carriers can be liquids, such as water and oils, including those of petroleum, animal, vegetable or synthetic origin, such as peanut oil, soybean oil, mineral oil, sesame oil and the like. The carriers can be saline, gum acacia, gelatin, starch paste, talc, keratin, colloidal silica, urea, and the like. In addition, auxiliary, stabilizing, thickening, lubricating and coloring agents can be used. In one embodiment, when administered to an animal, the antibody or compositions and pharmaceutically acceptable carriers are sterile. Water is a preferred carrier where the drug antibody conjugates are administered intravenously. Saline solutions and aqueous dextrose and glycerol solutions can also be employed as liquid carriers, particularly for injectable solutions. In one embodiment, the carrier includes excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents.

The pharmaceutical composition can be in the form of a liquid, e.g., a solution, syrup, solution, emulsion or suspension. The liquid can be useful for oral administration or for delivery by injection, infusion (e.g., IV infusion) or sub-cutaneously.

When intended for oral administration, the composition can be in solid or liquid form, where semi-solid, semi-liquid, suspension and gel forms are included within the forms considered herein as either solid or liquid.

As a solid composition for oral administration, the composition can be formulated into a powder, granule, compressed tablet, pill, capsule, chewing gum, wafer or the like form. Such a solid composition typically contains one or more inert diluents. In addition, one or more of the following can be present: binders such as carboxymethylcellulose, ethyl cellulose, microcrystalline cellulose, or gelatin; excipients such as starch, lactose or dextrins, disintegrating agents such as alginic acid, sodium alginate, corn starch and the like; lubricants such as magnesium stearate; glidants such as colloidal silicon dioxide; sweetening agents such as sucrose or saccharin; a flavoring agent such as peppermint, methyl salicylate or orange flavoring; and a coloring agent. When the composition is in the form of a capsule (e. g. a gelatin capsule), it can contain, in addition to materials of the above type, a liquid carrier such as polyethylene glycol, cyclodextrin or a fatty oil.

When intended for oral administration, a composition can comprise one or more of a sweetening agent, preservatives, dye/colorant and flavor enhancer. In a composition for administration by injection, one or more of a surfactant, preservative, wetting agent, dispersing agent, suspending agent, buffer, stabilizer and isotonic agent can also be included. Compositions can take the form of one or more dosage units.

Disclosed herein but not forming part of the invention is a method for treating a disease in a subject selected from a cancer, an immune disease, neurological disease, inflammatory disease, allergy, transplant rejection, viral infection, immune deficiency, and other immune system-related disease comprising administering an effective amount of an antibody or fragment thereof or a pharmaceutical composition as described herein to a subject in need thereof.

We also provide an antibody or fragment thereof as described above or a pharmaceutical composition as described above for use in the treatment of a disease, for example cancer, immune disease, neurological disease, inflammatory disease, allergy, transplant rejection, viral infection, immune deficiency, and other immune system-related disease cancer.

Disclosed herein but not forming part of the invention is an antibody or fragment thereof as described above or a pharmaceutical composition as described above for use in the manufacture for of a medicament for the treatment of a disease, for example cancer, immune disease, neurological disease, inflammatory disease, allergy, transplant rejection, viral infection, immune deficiency, and other immune system-related disease cancer.

As used herein, "treat", "treating" or "treatment" means inhibiting or relieving a disease or disease. For example, treatment can include a postponement of development of the symptoms associated with a disease or disease, and/or a reduction in the severity of such symptoms that will, or are expected, to develop with said disease. The terms include ameliorating existing symptoms, preventing additional symptoms, and ameliorating or preventing the underlying causes of such symptoms. Thus, the terms denote that a beneficial result is being conferred on at least some of the mammals, e.g., human patients, being treated. Many medical treatments are effective for some, but not all, patients that undergo the treatment.

The term "subject" or "patient" refers to an animal which is the object of treatment, observation, or experiment. By way of example only, a subject includes, but is not limited to, a mammal, including, but not limited to, a human or a non-human mammal, such as a non-human primate, murine, bovine, equine, canine, ovine, or feline.

As used herein, the term "effective amount" means an amount of an antibody, that when administered alone or in combination with an additional therapeutic agent to a cell, tissue, or subject, is effective to achieve the desired therapeutic or prophylactic effect under the conditions of administration.

In one embodiment, the disease is cancer. The cancer can be selected from a solid or non-solid tumor. For example, the cancer may be selected from bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, breast cancer, brain cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, kidney cancer, sarcoma of soft tissue, cancer of the urethra, cancer of the bladder, renal cancer, lung cancer, non-small cell lung cancer, thymoma, prostate cancer, mesothelioma, adrenocortical carcinoma, lymphomas, such as such as Hodgkin's disease, non-Hodgkin's, gastric cancer, leukemias such as ALL, CLL, AML, urothelial carcinoma leukemia and multiple myelomas.

In one embodiment, the tumor is a solid tumor. Examples of solid tumors which may be accordingly treated include breast carcinoma, lung carcinoma, colorectal carcinoma, pancreatic carcinoma, glioma and lymphoma. Some examples of such tumors include epidermoid tumors, squamous tumors, such as head and neck tumors, colorectal tumors, prostate tumors, breast tumors, lung tumors, including small cell and non-small cell lung tumors, pancreatic tumors, thyroid tumors, ovarian tumors, and liver tumors. Other examples include Kaposi's sarcoma, CNS, neoplasms, neuroblastomas, capillary hemangioblastomas, meningiomas and cerebral metastases, melanoma, gastrointestinal and renal carcinomas and sarcomas, rhabdomyosarcoma, glioblastoma, preferably glioblastoma multiforme, and leiomyosarcoma. Examples of vascularized skin cancers for which the antagonists of this invention are effective include squamous cell carcinoma, basal cell carcinoma and skin cancers that can be treated by suppressing the growth of malignant keratinocytes, such as human malignant keratinocytes.

In one embodiment, the tumor is a non-solid tumor. Examples of non-solid tumors include leukemia, multiple myeloma and lymphoma.

In one embodiment, the cancer is locally advanced unresectable, metastatic, or recurrent cancer. In one embodiment, the cancer has progressed after another treatment, for example chemotherapy.

Cancers include those whose growth may be inhibited using the antibodies of the invention include cancers typically responsive to immunotherapy. Non-limiting examples of preferred cancers for treatment include melanoma (e.g., metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer).

The immune disease can be selected from graft vs. host disease, arthritis, alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Meniere's disease, mixed connective tissue disease, multiple sclerosis, Neuromyelitis optica (NMO), type 1 or immune -mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, rheumatoid arthritis, sarcoidosis, scleroderma, Sjogren's syndrome, stiff-man syndrome, systemic lupus erythematosus, lupus erythematosus, takayasu arteritis, temporal arteristis/ giant cell arteritis, transverse myelitis, ulcerative colitis, uveitis, vasculitides such as dermatitis herpetiformis vasculitis, vitiligo, and Wegener's granulomatosis.

In one embodiment, the antibody, antibody fragment or pharmaceutical composition described herein is used in combination with an existing therapy or therapeutic agent, for example an anti-cancer therapy. The anti-cancer therapy may include a therapeutic agent or radiation therapy and includes gene therapy, viral therapy, RNA therapy bone marrow transplantation, nanotherapy, targeted anti-cancer therapies or oncolytic drugs. Examples of other therapeutic agents include other checkpoint inhibitors, antineoplastic agents, immunogenic agents, attenuated cancerous cells, tumor antigens, antigen presenting cells such as dendritic cells pulsed with tumor-derived antigen or nucleic acids, immune stimulating cytokines (e.g., IL-2, IFNa2, GM-CSF), targeted small molecules and biological molecules (such as components of signal transduction pathways, e.g. modulators of tyrosine kinases and inhibitors of receptor tyrosine kinases, and agents that bind to tumor- specific antigens, including EGFR antagonists), an anti-inflammatory agent, a cytotoxic agent, a radiotoxic agent, or an immunosuppressive agent and cells transfected with a gene encoding an immune stimulating cytokine (e.g., GM-CSF), chemotherapy. In one embodiment, the antibody is used in combination with surgery.

In one embodiment, we provide a photoactive antibody drug conjugate comprising an antibody described herein wherein the moiety that is conjugated is a toxic payload. Such conjugate can be used to kill tumor cells by antibody cell targeting upon light activation, internalization by the cells (generally cancerous), and payload release to expose the highly potent cytotoxic drugs to the tumor for toxin-mediated tumor cell killing. The toxic payload may be selected from small molecules (e.g. maytansanoid, auristatin), a protein toxin (e.g. Pseudomonas exotoxin, diphtheria toxin), a cytolytic immunomodulatory protein (e.g. Fas ligand) to kill targeted cells, a biologically active peptide (e.g. GLP-1) to extend the pharmacological half-life of the natural peptide, an enzymes (e.g. urease) to modify the biochemistry of the targeted microenvironment or radionuclides (e.g. 90Y, 111In) for either killing or imaging of tumor cells.

The toxic pay load may be conjugated to the antibody using a number of chemical linkers including cleavable linkers such as disulfides, hydrazones, peptides, or non-cleavable linkers such as thioethers. In an embodiment the toxic payload is conjugated to the antibody by an unnatural amino acid. In this embodiment a further unnatural amino acid is site specifically genetically incorporated into the antibody sequence, this unnatural amino acid may be different to the photocaged amino acid. The unnatural amino acid for attaching the toxic payload may be positioned away from the antigen binding portion of the antibody, it may be present at a site within the constant region or the Fc region of the antibody. Further there maybe multiple attachment sites for the toxic payload each comprising an unnatural amino acid which may be the same or different.

The unnatural amino acids which may be used to attach the toxic payload include *p-*acetylphenylalanine, alkyne lysine, *p*-azidol-_{L}-phenylalanine, *p*-azidol-_{L}-methylphenylalanine N6-((2-azidoethoxy)carbonyl)-_{L}-lysine. The carbonyl group of *p*-acetylphenylalanine can react with alkoxyamine-functionalised linkers to produce oxime-conjugated ADCs. The alkyne group of alkyne lysine can react with azide functionalised linkers through copper-catalysed Huisgen cycloaddition ("click" reaction). The azide groups of *p*-azidomethyl-_{L}-phenylalanine, *N*6-((2-azidoethoxy)carbonyl)-_{L}-lysine can react with alkyne-functionalized linkers through the copper-catalyzed Huisgen cycloaddition ("click" reaction).

In one embodiment, the antibody or composition is administered concurrently with a chemotherapeutic agent or with radiation therapy. In another specific embodiment, the chemotherapeutic agent or radiation therapy is administered prior or subsequent to administration of the composition disclosed herein, preferably at least an hour, five hours, 12 hours, a day, a week, a month, more preferably several months (e. g. up to three months), prior or subsequent to administration of composition of the present invention.

The antibody, antibody fragment or pharmaceutical composition described herein can be administered by any convenient route, including but not limited to oral, topical, parenteral, sublingual, rectal, vaginal, ocular, intranasal, pulmonary, intradermal, intravitreal, intratumoural, intramuscular, intraperitoneal, intravenous, subcutaneous, intracerebral, transdermal, transmucosal, by inhalation, or topical, particularly to the ears, nose, eyes, or skin or by inhalation. In another embodiment, delivery is of the nucleic acid encoding the drug, e.g. a nucleic acid encoding the molecule of the invention is delivered.

Parenteral administration includes, for example, intravenous, intramuscular, intraarterial, intraperitoneal, intranasal, rectal, intravesical, intradermal, topical or subcutaneous administration. Preferably, the compositions are administered parenterally.

In some embodiments, it can be desirable to administer the composition locally to the area in need of treatment, for example the tumor tissue, or by intravenous injection, infusion or topical administration. Such selective activation near the tumor site can lead to fewer side effects compared to conventional therapy based on antibody therapeutics. In this way, potentially higher dosages can be employed for the treatment.

The amount of the composition described herein that is effective/active in the treatment of a particular disease or condition will depend on the nature of the disease or condition, and can be determined by standard clinical techniques. In addition, in vitro or in vivo assays can optionally be employed to help identify optimal dosage ranges. The precise dose to be employed in the compositions will also depend on the route of administration, and the seriousness of the disease or disease, and should be decided according to the judgment of the practitioner and each subject's circumstances. Factors like age, body weight, sex, diet, time of administration, rate of excretion, condition of the host, drug combinations, reaction sensitivities and severity of the disease shall be taken into account.

Typically, the amount is at least about 0.01% of an antibody by weight of the composition. When intended for oral administration, this amount can be varied to range from about 0.1 % to about 80% by weight of the composition. Preferred oral compositions can comprise from about 4% to about 50% of the antibody of the present invention by weight of the composition.

Compositions can be prepared so that a parenteral dosage unit contains from about 0.01 % to about 2% by weight of the antibody. For administration by injection, the composition can comprise from about typically about 0.1 mg/kg to about 250 mg/kg of the animal's body weight, preferably, between about 0.1 mg/kg and about 20 mg/kg of the animal's body weight, and more preferably about 1 mg/kg to about 10 mg/kg of the animal's body weight. In one embodiment, the composition is administered at a dose of about 1 to 30 mg/kg, e.g., about 5 to 25 mg/kg, about 10 to 20 mg/kg, about 1 to 5 mg/kg, or about 3 mg/kg. The dosing schedule can vary from e.g., once a week to once every 2, 3, or 4 weeks.

In one embodiment, the method comprises activating the antibody or fragment thereof by irradiating a target tissue or a subject. As used herein, "activating" means that due to expose to light, the caging group is removed from the antibody and the antibody is now free to bind to the target antigen. The wavelength is chosen dependent on the photocaging group used. For example, it can be 365nm. In another embodiment, the light is in the infrared region of the light spectra. For example, when used in combination with upconverting nanoparticles, the photocage group can be decaged using 975 nm light, in the Infrared region of the light spectra.

Disclosed herein, not forming part of the invention is an in vitro or in vivo method for diagnosing a disease comprising administering to a subject an antibody or fragment thereof as disclosed herein wherein said antibody is conjugated to a label. The method further comprises activating the antibody or fragment thereof by irradiating a target tissue or a subject.

We also provide an in vitro method of reducing tumor cell growth and/or proliferation in a, tumour cell, tissue or cell culture,as set out in the claims, comprising administering a effective amount of an antibody, antibody fragment or pharmaceutical composition described herein.

In one embodiment, we provide an in vitro method of reducing tumor cell growth and/or proliferation in a, tumour cell, tissue or cell culture comprising
a) contacting a tumor cell with an effective amount of an antibody, antibody fragment or pharmaceutical composition described herein, wherein the antibody targets an antigen present on the surface of a tumor cell and exerts an inhibitory effect on growth and/or proliferation of the tumor cell when bound to the antigen;
b) irradiating the antibody or fragment with a light source to release the photocaging group and induce specific binding of the antibody to the target antigen; and
thereby inhibiting growth and/or proliferation of the tumor cell.

In one embodiment, we provide an in vitro method of reducing tumor cell growth and/or proliferation in a tumour cell, tissue or cell culture comprising
a) contacting a tumor cell with an effective amount of an antibody, antibody fragment or pharmaceutical composition described herein, wherein the antibody that targets an antigen present on the surface of a tumor cell is internalised when bound to the antige; and
b) irradiating the antibody or fragment with a light source to release the photocaging group and induce specific binding of the antibody to the target antigen wherein said antibody and is internalised upon binding to the antigen; and
thereby killing the cell.

We also provide an in vitro method of killing a cell, as set out in the claims, comprising administering a therapeutically effective amount of an antibody, antibody fragment or pharmaceutical composition described herein. In one embodiment, we provide a method of killing a cell comprising:
a) contacting a cell comprising a cell surface protein with a therapeutically effective amount of an antibody, antibody fragment or pharmaceutical composition described herein, wherein the antibody targets an antigen present on the surface of a tumor cell is internalised when bound to the antigen;
b) irradiating the antibody or fragment with a light source to release the photocaging group and induce specific binding of the antibody to the target antigen wherein said antibody is internalised upon binding to the antigen; and
c) killing the cell.

In the methods above, irradiating the antibody or fragment comprises irradiating the cell, tissue or cell culture; and/or irradiating a tumor in the subject, cell, tissue or cell culture.

Disclosed herein, not forming part of the invention, is a method for light dependent delivery of cytotoxic drugs to a cell, tissue or subject comprising providing an antibody, antibody fragment or pharmaceutical composition described herein conjugated to a toxic payload. In one embodiment, we provide a method for light dependent delivery of cytotoxic drugs comprising:
a) contacting a cell or tissue an antibody, antibody fragment or pharmaceutical composition described herein wherein said antibody or fragment is conjugated to a toxic payload, wherein the antibody targets an antigen present on the surface of a tumor cell is internalised when bound to the antigen;
b) irradiating the antibody or fragment to release the photocaging group and induce specific binding of the antibody to the target antigen wherein said antibody drug conjugate is internalised upon binding to the antigen; and
c) killing the cell.

The method may be an in vitro or in vivo method for application in a subject, cell, tissue or cell culture.

Disclosed herein, not forming part of the invention, is a method for imaging of tumor cells comprising providing an antibody, antibody fragment conjugated to a label and delivering said antibody or fragment to a target cell or tissue. The method may be an in vitro or in vivo method for application in a subject, cell, tissue or cell culture.

Disclosed herein, not forming part of the invention, is a method of reducing the side effects associated with targeting cancer and/or tumour cells, comprising administering to a subject in need thereof the antibody, antibody fragment or pharmaceutical composition described herein. The method further comprises irradiating the antibody or fragment to release the photocaging group and induce specific binding of the antibody to the target antigen.

The methods may comprise applying a suitable light source selected from the group consisting of a filtered conventional light source, a diode array, and a laser. For example, by using fiber- coupled laser diodes with diffuser tips, light, such as NIR, light can be delivered within several centimetres of otherwise inaccessible tumors located deep to the body surface. In addition to treating solid cancers, circulating tumor cells can be targeted since they can be excited when they traverse superficial vessels (for example using the NIR LED wearable devices disclosed herein).

Depending on the part of the body being treated, the photoactive antibodies can be either injected intravenously into the diseased area or applied topically to the skin.

We also disclose, but do not claim, a nucleic acid encoding an antibody or antibody fragment as described herein.

We also disclose, but do not claim, a vector comprising a nucleic acid encoding an antibody or antibody fragment as described herein. Furthermore, we provide an isolated nucleic acid construct comprising at least one nucleic acid as defined above. The construct may be in the form of a plasmid, vector, transcription or expression cassette. Thus, the invention also relates to a plasmid, vector, transcription or expression cassette comprising a nucleic acid of the invention.

Disclosed herein is an isolated recombinant host cell comprising one or more nucleic acid molecule plasmid, vector, transcription or expression cassette as described above. The host cell may be a bacterial, viral, plant, fungal, mammalian or other suitable host cell. In one embodiment, the cell is an E. coli cell. In another embodiment, the cell is a yeast cell. In another embodiment, the cell is a Chinese Hamster Ovary (CHO) cell, HeLa cell or other cell that would be apparent to the skilled person.

We also provide a kit comprising the antibody or fragment thereof as described herein. The kit may comprise a LED wearable device or an LED implantable device. A LED wearable device devices can include an article of clothing, jewellery, or a covering, and a near infrared (NIR) light emitting diode (LED) that is incorporated into the article of clothing, jewellery, or covering. Such devices can further include power and/or cooling sources. This permits the patient to wear the device (or be covered by the device) for extended periods of time, thus permitting treatment of tumor cells present in the blood or circulatory system.

We also provide a method for producing an antibody or antibody fragment as set out in the claims, comprising identifying an amino acid residue in the antigen binding region residue for site specific modification and introducing a photocaged group. The method further comprises expressing the antibody in a host cell and isolating the antibody from the host cell.

Unless otherwise defined herein, scientific and technical terms used in connection with the present disclosure shall have the meanings that are commonly understood by those of ordinary skill in the art.

The term "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein. Unless context dictates otherwise, the descriptions and definitions of the features set out above are not limited to any particular aspect or embodiment of the invention and apply equally to all aspects and embodiments which are described.

The invention is further described in the non-limiting examples.

### Examples

Here, we demonstrate efficient site-specific incorporation of several unnatural amino acids with different chemical groups, including tert-butoxycarbonyl (Boc), alkyne, azide, and o-nitrobenzyl (photocaging) groups into 7D12. Using a western blot-based approach on the surface of cancer cells, we show that the presence of a photocaging group at specific tyrosine residues in the antigen binding region of 7D12 inhibits its binding to EGFR and the binding is restored upon irradiation with 365 nm light. The interaction of 7D12 and its photocaged mutants with EGFR was investigated using molecular dynamics (MD) simulations to gain atomic level understanding of the effect of photocaging group on 7D12-EGFR interaction. We also show that photoactive antibodies can mediate delivery of small molecule fluorophores to the surface of EGFR-positive live cancer cells in a light-dependent manner.

### Example 1: Efficient genetic site-specific incorporation of photocaged tyrosine into antibody fragments

Several suppressor plasmids have been employed in different studies for unnatural amino acid incorporation into proteins expressed in E. coli. These plasmids contain orthogonal aminoacyl-tRNA synthetase (aaRS)/ tRNA pairs that incorporate unnatural amino acids in response to an amber stop codon or a quadruplet codon. The suppressor plasmids vary in their origin of replication, promotors that drive the expression of aaRS and tRNA, and the copy number of aaRS and tRNA gene. Hence, we screened five reported suppressor plasmids, with the aim of developing an optimized system for unnatural amino acid incorporation into antibody fragments.

First, wild-type 7D12 (wt7D12) was cloned into pSANG10 plasmid forming pSANG10_7D12 plasmid; pSANG10 has earlier been employed for efficient expression of single chain antibody fragments in the periplasm of E. coli (Martin, C. D.; Rojas, G.; Mitchell, J. N.; Vincent, K. J.; Wu, J.; McCafferty, J.; Schofield, D. J., A simple vector system to improve performance and utilisation of recombinant antibodies. BMC Biotechnol. 2006, 6, 46.) and (Biffi, G.; Tannahill, D.; McCafferty, J.; Balasubramanian, S., Quantitative visualization of DNA G-quadruplex structures in human cells. Nat. Chem. 2013, 5 (3), 182-6). Using pSANG10_7D12, we obtained a yield of 10.1 mg of wt7D12 per litre of culture after purification. Next, we optimized site-specific incorporation of unnatural amino acids into 7D12. Mutants of Methanococcus jannaschii Tyrosyl-tRNA synthetase (MjTyrRS)/MjtRNA pair and Methanosarcina Pyrrolysyl-tRNA synthetase (PyIRS)/tRNA pair have been employed to genetically encode several unnatural amino acids in E. coli. We screened suppressor plasmids containing these two aaRS/ tRNA pairs and compatible with the pSANG10_7D12 plasmid: pSUP_MjCNFRS/6xMjtRNA (Ryu, Y.; Schultz, P. G., Efficient incorporation of unnatural amino acids into proteins in Escherichia coli. Nat. Methods 2006, 3 (4), 263-5) pEvolv_2xMjCNFRS/ MjtRNA) (Young, T. S.; Ahmad, I.; Yin, J. A.; Schultz, P. G., An enhanced system for unnatural amino acid mutagenesis in E. coli. J. Mol. Biol. 2010, 395 (2), 361-74) and pULTRA_MjCNFRS/MjtRNA35 plasmids for incorporation of 4-azido-L-phenylalanine, and pCDF_3xPyIRS/ PyltRNA (Wang, K.; Sachdeva, A.; Cox, D. J.; Wilf, N. M.; Lang, K.; Wallace, S.; Mehl, R. A.; Chin, J. W., Optimized orthogonal translation of unnatural amino acids enables spontaneous protein double-labelling and FRET. Nat. Chem. 2014, 6 (5), 393-403) and pULTRA_PyIRS/PyltRNA plasmids for incorporation of N6-(tert-butoxycarbonyl)-L-lysine and N6-[(2-propynyloxy)carbonyl]-L-lysine in 7D12. pULTRA_MjCNFRS/MjtRNA plasmid is most efficient at genetic incorporation of 4-azido-L-phenylalanine. We thus employed the pULTRA plasmid for incorporation of photocaged tyrosine (pcY) in 7D12, after replacing the MjCNFRS (aaRS evolved for 4-cyano-L-phenylalanine) with MjpcYRS (aaRS evolved for pcY). We obtained near-wild-type level expression of 7D12 containing site-specifically incorporated pcY at position 32 using the pULTRA_MjpcYRS/MjtRNA plasmid. Subsequently, we replaced the MjCNFRS/MjtRNA in pULTRA plasmid with PyIRS/ PyltRNA pair, for incorporation of N6-(tert-butoxycarbonyl)-L-lysine and N6-[(2-propynyloxy)carbonyl]-L-lysine. The pULTRA plasmid is relatively inefficient at incorporation of these unnatural amino acids in 7D12, which might be due to differences in the charging efficiency of MjRS/MjtRNA and PyIRS/tRNA pairs. However, these differences in the charging efficiency can be compensated by using a plasmid with higher copies of aaRS and we observe that pCDF plasmid with three copies of PyIRS, pCDF (3xPyIRS/PyltRNA), shows significantly improved incorporation of N6-(tert-butoxycarbonyl)-L-lysine and N6-[(2-propynyloxy)carbonyl]-L-lysine in 7D12.

Upon examining the crystal structure of 7D12 complexed with domain III of EGFR (PDB ID: 4KRL) (Schmitz, K. R.; Bagchi, A.; Roovers, R. C.; van Bergen en Henegouwen, P. M.; Ferguson, K. M., Structural evaluation of EGFR inhibition mechanisms for nanobodies/VHH domains. Structure 2013, 21 (7), 1214-24) we identified three tyrosine residues in the antigen binding site of 7D12, viz. Y32, Y109 and Y113, as candidates for developing photocaged mutants. These tyrosine residues were replaced with pcY by assigning amber stop codon (TAG) to these positions, forming mutants, 7D12pcY32, 7D12pcY109, and 7D12pcY113, respectively. wt7D12 and the three amber mutants were expressed using cells containing pULTRA_MjpcYRS/MjtRNACUA (that directs site-specific incorporation of pcY) and pSANG10_7D12, pSANG10_7D12_32TAG, pSANG10_7D12_109TAG, or pSANG10_7D12_113TAG plasmids (that provide the gene for periplasmic expression of 7D12 and its amber mutants). The protein expression was performed both in the presence and absence of pcY, and the expression of full-length protein was dependent on the addition of pcY for the amber mutants. This is shown in Figure 1A. The yield of 7D12pcY32, 7D12pcY109, and 7D12pcY113 after purification were 5.3, 3.2, 1.7 mg per litre of culture, respectively. Site-specific incorporation of pcY in 7D12 was further confirmed by Electrospray Ionization Mass Spectrometry (ESI-MS). This is shown in Figure 1B.

### Example 2: Assessing the binding of photocaged antibodies to EGFR on the surface of cancer cells

Several methods exist for measuring the interaction of proteins in solution, including enzyme-linked immunosorbent assay (ELISA), surface plasmon resonance (SPR), isothermal titration calorimetry (ITC) and dynamic light scattering (DLS) . However, these methods often require purified proteins and measure the binding of biomolecules in non-cellular environments. Methods have also been developed to measure the binding of biomolecules on the cell surface using imaging SPR and on live cells using flow cytometry. These methods require and/or employ sophisticated instruments. Labelled proteins have also been used for studying biomolecular interaction. However, labelling of proteins with probes can often influence their interaction with the target.

In the present investigation, an easily accessible and economical approach was adopted to assess protein-protein binding by performing western blot on the cell surface in the following manner. A431 cells, that are human epidermal carcinoma cells with high levels of EGFR on their cell surface, were plated in a 96-well plate, incubated with 7D12 (or its photocaged mutants) containing a C-terminus hexa-histidine tag (His6), and then washed to remove unbound 7D12. Cells were then fixed to the surface of the wells. A primary antibody specific to His6 was added after incubation of the cells with blocking buffer. This was followed by incubation with Horse Radish Peroxidase (HRP)-linked secondary antibody that binds to the primary antibody. After adding the substrate for HRP, cells were imaged for chemiluminescence. This is shown in Figure 2A. This approach does not require purified EGFR and assesses the binding of antibody to the extracellular domain of EGFR on cell surface. The technique is similar to on-cell western and cell-ELISA used for quantification of cell surface antigens.

Prior to measuring the binding of photocaged mutants of 7D12, we used ESI-MS to confirm light-induced decaging of 7D12pcY32, 7D12pcY109, and 7D12pcY113. The molecular weight of all the pcY mutants was reduced to that of wt7D12 after irradiation with 365 nm light for 4 min, confirming the loss of o-nitrobenzyl group from tyrosine residues in the photocaged mutants. This is shown in Figure 2B.

In order to assess if the presence of pcY at positions 32, 109 and 113 in 7D12, inhibit 7D12-EGFR binding, western blot experiments were performed on the surface of A431 cells as described above. For the 7D12pcY32 and 7D12pcY113 mutants, near background level chemiluminescence signal was observed for up to 500 nM concentration. This is shown in Figure 2C, Figure 2D and 2E, demonstrating that the binding between 7D12 and EGFR is inhibited due to the presence of pcY at positions 32 and 113 in 7D12. The binding of these mutants was recovered by 86% and ~76%, respectively, upon irradiation with 365 nm light; demonstrating light-mediated activation of antibody-antigen binding. Interestingly, pcY at position 109, despite being at the binding interface, does not inhibit 7D12-EGFR binding. We explain these differences in the binding affinity of photocaged mutants using MD simulations, later in this study.

A series of control experiments were performed to demonstrate the viability of the western blot-based assay to study 7D12-EGFR binding on the cell surface. When 7D12 was incubated with an MDA-MB231, a cell line with low levels of EGFR, the chemiluminescence signal was significantly lower compared to the signal for A431 cancer cell line. This is shown in Figure 2F. This supports our premise that the observed chemiluminescence is due to specific interaction between 7D12 and EGFR, and not due to non-specific binding of 7D12 to the cell surface. We also measured the binding of an unrelated His6-tagged antibody fragment, RR6-VHH (Spinelli, S.; Frenken, L. G.; Hermans, P.; Verrips, T.; Brown, K.; Tegoni, M.; Cambillau, C., Camelid heavy-chain variable domains provide efficient combining sites to haptens. Biochemistry 2000, 39 (6), 1217-22), to A431 cells using this assay. Near-background level of chemiluminescence was observed with RR6-VHH, demonstrating that the observed signal is not due to the non-specific interaction of antibody fragments with the surface of A431 cancer cells. This is shown in Figure 2G.

### Example 3: Molecular dynamics simulations explain the effect of photocaged tyrosine on the 7D12- EGFR interaction

In the crystal structure of 7D12-EGFR domain III complex (PDB ID: 4KRL), Y32, Y109, and Y113 residues in 7D12 lie at the binding interface. Hence, in our experiments, we expected that substituting any of these tyrosine residues with pcY could inhibit or affect 7D12-EGFR binding. While two of the three mutants show expected behaviour, i.e., significantly reduced binding to EGFR, one of the mutants, 7D12pcY109, binds to EGFR with affinity comparable to wt7D12. We investigated this difference in binding behaviour through a description of 7D12-EGFR interactions and dynamics in the presence and absence of photocaging group, using MD simulations.

All-atom MD simulations were performed for four systems: complexes of EGFR domain III with wt7D12, 7D12pcY32, 7D12pcY109, and 7D12pcY113 in the presence of explicit water and ions. This is shown in Figure 3A. The crystal structure for 7D12-EGFR domain III complex (PDB ID: 4KRL) was used as the starting structure for wt7D12-EGFR domain III simulations, as well as to generate the three mutants, by modelling in pcY to substitute Y32, Y109, and Y113, respectively. On comparison of the dynamics of the four systems during 300ns simulations each, it emerged that wt7D12 and 7D12pcY109 remain bound to EGFR, while 7D12pcY32 and 7D12pcY113 show unbinding from EGFR for prolonged periods. This is shown in Figure 3A-3C. Monitoring the root mean square deviation (RMSD) from starting conformation, the distance between residues R30 (in 7D12) and D355 (in EGFR), known to form a key salt bridge for 7D12-EGFR binding (Schmitz, K. R.; Bagchi, A.; Roovers, R. C.; van Bergen en Henegouwen, P. M.; Ferguson, K. M., Structural evaluation of EGFR inhibition mechanisms for nanobodies/VHH domains. Structure 2013, 21 (7), 1214-24), and the number of contacts formed by 7D12 to EGFR during the simulations, all show trends consistent with the stable binding of wt7D12 and 7D12pcY109 to EGFR, while the complexes with 7D12pcY32 and 7D12pcY113 mutants are unstable. This is shown in Figure 3C.

On inspecting the wt7D12-EGFR simulations for interactions formed by Y32 and Y113 in 7D12 with residues in EGFR, it was seen that both form some non-specific interactions, mainly with L325 on EGFR. As such, Y32 and Y113 do not appear to show any notable hydrogen bonding or packing interactions with residues in EGFR during the simulations. However, upon substitution by pcY, in both cases, the additional o-nitrobenzyl group protrudes into the binding interface, contacting several EGFR residues. Although initially appearing to be accommodated at the binding interface, as the simulation proceeds, it does not form stable contacts that could compensate for the crowding it causes in this region, and eventually, binding is disrupted. On examining the behaviour of Y109, which is also at the binding interface, it is seen to prefer remaining oriented nearly parallel to the EGFR surface throughout the simulation of wt7D12-EGFR complex, interacting significantly with only S418 on EGFR. The simulation of 7D12pcY109-EGFR complex demonstrates that the additional o-nitrobenzyl group is accommodated in a large solvent-accessible cleft and does not cause a steric clash with any residue in EGFR (Figure 3A-C)- allowing the complex to remain bound.

### Example 4: Delivery of cargo to live EGFR-positive cancer cells in a light-dependent manner

Antibodies and antibody fragments have been used earlier for delivery of fluorophores and cytotoxic drugs to cancer cells. We designed experiments to examine if photoactive antibodies can deliver fluorophores to the surface of live A431 cells in a light-dependent manner. 7D12 and 7D12pcY32 were labelled using N-hydroxysuccinimide (NHS) ester of a fluorophore, BODIPY-TMR-X. We first assessed if the presence of this label on 7D12 influences 7D12-EGFR binding, using western blot experiments. Comparison of unlabelled and labelled 7D12 reveal that the binding is reduced by ~1.5-fold due to the presence of the BODIPY-TMR-X label; hence at least 1.5-fold higher concentration of labelled sample would be required for further experiments. This is shown in Figure 4. Furthermore, to ensure a detectable fluorescence signal, a high concentration of 500 nM labelled 7D12 was used for microscopy experiments.

The light-dependent localization of photoactive antibody on the surface of live A431 cells was evaluated using fluorescence microscopy. The microscope was fitted with a flow chamber. The flow rate of media and the labelled antibody fragments was fixed at 1 ml/min throughout the experiment. Images were acquired every 30 sec, except during irradiation with 365 nm light. Media was initially passed for 5 min through the chamber containing A431 cells, followed by 500 nM solution of labelled 7D12pcY32 for 2 min, and then again media for 10 min. 30 sec after injecting the labelled 7D12pcY32, a burst of fluorescence was observed, which continued for 2 min during the movement of labelled 7D12pcY32 over the cells. However, the signal reverts to background level in less than 1.5 min after the flow of labelled 7D12pcY32 was stopped, indicating that 7D12pcY32 does not bind to the surface of A431 cells. This is shown in Figure 5A. Next, labelled 7D12pcY32 was again passed through the chamber for 2 min, but this time, after 1 min, the chamber was irradiated with 365 nm light for 1 min. Subsequent to an initial burst of fluorescence due to movement of labelled 7D12pcY32, a detectable fluorescence signal was observed even 2 min after stopping the injection of labelled 7D12pcY32. Comparison of fluorescence signals observed 1.5 min after stopping the flow of labelled 7D12pcY32 before (shown in Figure 5A) and after (shown in Figure 5C) irradiation, demonstrates light-dependent delivery of fluorophores mediated by 7D12 on the surface of live A431 cells. The fluorescence signal from 7D12 bound to the surface of A431 cells eventually decays to background level, presumably because of endocytosis of 7D12 and degradation of the fluorophore (shown in Figure 5D).

Finally, as a control, 500 nM solution of labelled wt7D12 is passed through the chamber for 2 min and fluorescence signal was observed 1.5 mins after stopping the flow of labelled wt7D12, consistent with receptor-mediated localization of wt7D12 (shown in Figure 5E). The fluorescence signal for wt7D12 is stronger than for decaged 7D12pcY32 (shown in Figure 5C) as the latter flows in the caged form for 1 min and decaged form for 1 min, thus reducing the effective concentration of actively binding 7D12 to half, when compared to wt7D12 that flows for 2 min. Overall, these results are consistent with light-dependent delivery of fluorophores on the surface of EGFR-positive cancer cells.

We report highly efficient genetic site-specific incorporation of several unnatural amino acids into antibody fragments expressed in E. coli. Site-specific incorporation of photocaged amino acid into the antigen binding domain of antibody allowed development of photoactive antibodies. We employed a simple western blot-based approach to assess the interaction between antibody and its antigen on the surface of cancer cells, and demonstrated that the binding of antibody to its corresponding antigen can be controlled by light. MD simulations were used to study the dynamics of 7D12-EGFR interaction that explain the effect of the photocaging group when placed at different sites in the 7D12-EGFR binding interface. These studies also pave the way for potential use of MD simulations to predict candidate residues for photocaging in different proteins. We also used photoactive antibodies to deliver small molecule fluorophores on the surface of live cancer cells in a light-dependent manner. We anticipate that the methods and the photoactive antibodies developed in this study will find applications in several fields, including medicine, nanotechnology and biotechnology, and encourage the development of other photoactive biotherapeutics.

### Example 5: Homogeneously labelled photoactive antibody-drug conjugates (ADCs)

ADCs are an important class of immunotherapeutics used in cancer therapy that have two components: a) an antibody that binds to cell surface receptors on cancer cells, and b) a cytotoxic drug that kills the cancer cells. However, most ADC's have side effects due to binding of antibody to healthy cells. Development of ADC's that can be activated at the site tumour using light will reduce the side effect of such immunotherapeutics. Towards this goal, we have optimised site-specific incorporation of photocaged tyrosine and alkyne lysine (a bio-orthogonal function group) into antibody fragment 7D12 (Figure 6). Identity of full-length 7D12 containing photocaged tyrosine and alkyne lysine has been confirmed by mass spectrometry. In subsequent experiments, the alkyne-containing amino acid that will be conjugated to a cytotoxic drug molecule, monomethyl auristatin E (MMAE), resulting in homogeneously labelled photoactive ADC. To the best of our knowledge, this would be the first example of a homogeneously labelled light-activated antibody drug conjugate that rely on controlling the direct binding of antibody to its corresponding antigen using light. It may be noted that antibodies linked to light-activated small molecule drugs have been developed earlier (Nature Medicine (2011) Vol. 17, Pg. 1685-1691). However, such antibodies would still be able to bind to healthy cells independent of light and cause undesired cytotoxicity on healthy cells due to such binding. Controlling the direct binding of ADC to its corresponding antigen using light, at the site of cancer cells, can thus minimize the side effects of ADCs.

### Materials and methods

### Unnatural amino acids and cell lines

p-Azido-L-phenylalanine and N6-(tert-butoxycarbonyl)-L-lysine were purchased from BACHEM. O-(2-Nitrobenzyl)-L-tyrosine and N6-[(2-propynyloxy)carbonyl]-L-lysine were synthesized using a procedure similar to reported earlier (Deiters, A.; Groff, D.; Ryu, Y.; Xie, J.; Schultz, P. G., A genetically encoded photocaged tyrosine. Angew. Chem. Int. Ed. Engl. 2006, 45 (17), 2728-31) and (Nguyen, D. P.; Lusic, H.; Neumann, H.; Kapadnis, P. B.; Deiters, A.; Chin, J. W., Genetic encoding and labeling of aliphatic azides and alkynes in recombinant proteins via a pyrrolysyl-tRNA Synthetase/tRNA(CUA) pair and click chemistry. J. Am. Chem. Soc. 2009, 131 (25), 8720-8721). Epithelial squamous carcinoma cell line, A-431, and human breast adenocarcinoma cell line, MDA-MB-231, were purchased from Sigma-Aldrich. All mammalian cell lines were cultured in DMEM (Gibco, Invitrogen) containing L-glutamine, 4.5 g/L D-Glucose, 110 mg/L Sodium pyruvate, 10% (v/v) foetal bovine serum (FBS), and a cocktail of penicillin and streptomycin (PEN/STREP). This medium will be referred to as "complete medium" hereafter. Mammalian cells were grown in humidified atmosphere in 5% CO2 at 37 °C.

### Expression of wt7D12, 7D12pcY32, 7D12pcY109, and 7D12pcY113

Chemically competent BL21(DE3)pLysS cells containing pULTRA_MjpcYRS/MjtRNACUA plasmid were transformed with pSANG10_7D12 plasmid or its amber mutants. The cells were recovered by incubating in 1 ml SOB medium at 37oC for 1h. 50 µl of recovered cells were transferred on LB agar plate supplemented with 50 µg ml-1 of kanamycin and 75 µg ml-1 of spectinomycin. The plates were incubated at 37°C for 12-16 hrs. A single colony from each plate was used for inoculation of 50 ml of 2xTY-GKS media (2xTY media supplemented with 4% glucose, 50 µg ml-1 kanamycin and 75 µg ml-1 of spectinomycin) and incubated overnight (12-16 h) at 37°C, 220 rpm. The overnight culture was diluted to OD600= 0.1 and incubated until the OD600 reached 0.4-0.6 (37°C, 220rpm, 2-3 h). Once the desired growth was reached, the culture was induced with IPTG (1 mM final concentration) and supplemented with pcY (4 mM final concentration) for expression with unnatural amino acids. The culture was incubated overnight (30°C, 160 rpm, 12-16 h). Cells were pelleted (3200g, 4°C, 10 min) and periplasmic extraction was performed using standard methods. After periplasmic extraction, the protein was purified via its C-terminus hexa-histidine tag using Ni-NTA resin. Subsequently, the samples were concentrated using Vivaspin 500 columns with 3 kDa molecular weight cut-off (GE Healthcare) and the yields were determined using a calorimetric Pierce BCA protein assay (Thermo Fisher Scientific) measured at 562 nm. All protein samples were analyzed using SDS-PAGE. Samples were heated with Nu-PAGE LDS loading buffer (Invitrogen) at 95°C for 15 min, centrifuged at 13,000 g for 15 min at 4°C and loaded on 4-12% Bis-Tris gel (Invitrogen) along with BIO-RAD pre-stained protein ladder (Precision Plus All Blue Protein Standards) as a marker. The gel was stained with Coomassie Blue (InstantBlue, Expedeon). The identity of protein samples was further confirmed using electrospray ionization mass spectrometry coupled with liquid chromatography (LC-MS).

### Western blot on the surface of cancer cells for measuring the binding of His-tagged antibody fragments

A-431 and MDA-MB-231 cells were grown in a T-75 flask in complete medium (DMEM, 10% FBS, 1% PEN/STREP) using standard tissue culture procedures until 80-90% confluence. After washing with 1x phosphate buffered saline (PBS) and trypsinising, cells were pelleted (300g, 5 minutes) and resuspended in 10 ml fresh complete medium. The cells were then counted on a hemocytometer and diluted to 200,000 cells/ml. 200 µl of this solution was dispensed into each well (40,000 cells/well) of a 96-well plate and grown overnight until 80-90% confluence. Once the desired confluence was reached, medium was replaced with 200 µl of complete medium supplemented with 7D12 or its photocaged mutants at the desired concentration. The plate was incubated for 10 minutes (37°C, 5% CO2). After removing medium, the cells were fixed using formaldehyde. 150 µl of 3.7% formaldehyde solution in 1xPBS was added to each well and incubated for 20 min at room temperature. The formaldehyde solution was removed and cells were washed three times (200 µl, 5 minutes, gentle rocking) with PBST (1X PBS supplemented with 1% Tween-20). After removing the wash buffer, 100 µl of blocking buffer (10% milk in PBST) was added and cells were incubated at room temperature for 1 h with gentle rocking. The blocking buffer was removed. 50 µl solution containing primary anti-6x-His tag antibody was added to each well and the plate was incubated at room temperature for 1 h. The primary antibody solution contained mouse anti-6x-His tag antibody (Thermo Fisher Scientific) at 1:500 dilution and 1% milk in PBST. After incubation with the primary antibody, cells in each well were washed three times with PBST (200 µl, 5 minutes, gentle rocking). Subsequently, 50 µl of HRP-linked secondary antibody solution was applied to each well and incubated at room temperature for 1 h. The HRP-linked secondary antibody solution contained anti-mouse-HRP-linked antibody (Cell signaling) at 1:1000 dilution and 1% milk in PBST. After incubation with secondary antibody, the cells were washed five times with PBST (200 µl, 5 minutes, gentle rocking). Finally, 200 µl of SuperSignal chemiluminescent Substrate (Thermo Scientific) was added to each well and the plate was imaged using BIORAD GelDoc XR+. The plate was further quantified by measuring chemiluminescence signal using a CLARIOstar plate reader (BMG labtech).

### Computational methods

The crystal structure for 7D12-EGFR domain III complex (PDB ID: 4KRL) was used as the starting structure for wt7D12-EGFR domain III simulations. This crystal structure was also used to generate the three complexes with the mutants, 7D12pcY32, 7D12pcY109, and 7D12pcY113: pcY was modelled in to substitute Y32, Y109, and Y113, respectively. The CHARMM27 force field with CMAP corrections was adopted for proteins and ions, and CGENFF parameters were used for pcY. Parameter assignment and optimization for pcY, was done using ffTK, following the CGENFF methodology. Quantum mechanical target data for parameter optimization was generated using GAUSSIAN09. Using VMD, each of the four complexes was solvated with water molecules modelled as TIP3P, and Na+ and Cl- ions were added to neutralize the system with a net concentration of 150 mM. All the simulations were performed using NAMD 2.9. The systems were minimized for 10,000 steps with all heavy atoms of the protein restrained with a spring constant of k = 5 kcal/mol/Å2. The restraints on the side chains were then removed and the systems were again minimized for 10,000 steps, followed by equilibration for 0.2 ns using the NVT ensemble, with stepwise heating up to 310 K, to allow the side chains to relax. Next, all the restraints were removed and the systems were again minimized for 10000 steps, then equilibrated for 0.2 ns in the NVT ensemble with stepwise heating to 310K. Finally, the system was equilibrated in the NPT ensemble for 0.5 ns, followed by the data collection runs for 300 ns using the NPT ensemble and periodic boundary conditions. Temperature was maintained at 310 K using Langevin dynamics with a damping coefficient of 1 ps-1. Pressure was kept at 1 atm using the Nosé-Hoover Langevin piston method with a piston period of 100 fs and a piston decay of 50 fs. Short-range interactions were cut off at 12 Å with a switching applied at 10 Å. Long-range electrostatic forces were calculated using the particle mesh Ewald (PME) method at a grid density of >1 Å-3. Bonded, non-bonded, and PME calculations were performed at 2-, 2-, and 4-fs intervals, respectively.

## Claims

1. An antibody or fragment thereof wherein said antibody or fragment comprises a photocaged amino acid in one of the CDRs of its antigen binding region, wherein: i) the photocaged amino acid comprises a light-removable protection group; ii) the photocaged amino acid is present at a site in which the photocaged amino acid inhibits interaction and/or binding of the antibody to its antigen, wherein binding and/or interaction of the antibody to its antigen can be induced upon activation with a light source, and wherein the antibody or fragment thereof is conjugated to another moiety.

2. The antibody or fragment thereof according to claim 1 wherein said amino acid includes a photoactive group selected from an o-nitrobenzyl functional group and variants thereof.

3. The antibody or fragment thereof according to claim 1 or 2 wherein said fragment comprises a F(ab')2, Fab, Fv, sFv scFv or dAbs.

4. The antibody or fragment thereof according to any one of the proceeding claims wherein said moiety is selected from a half life extension moiety, label or toxic moiety, optionally
wherein said moiety is attached via an unnatural amino acid.

5. The antibody or fragment thereof according to a preceding claim wherein said antibody or fragment thereof binds to a cell surface antigen.

6. The antibody or fragment thereof according to a preceding claim wherein said antibody or fragment thereof binds to a tumor associated antigen or an immune checkpoint molecule.

7. A pharmaceutical composition comprising an antibody or fragment thereof according to a preceding claim and optionally a pharmaceutically acceptable carrier.

8. An antibody or fragment thereof according to any of claims 1 to 6 or a pharmaceutical composition according claim 7 for use in the treatment of disease, wherein: i) the antibody or fragment thereof is to be activated by irradiating a target tissue with a light source, optionally wherein the wavelength is 365nm; ii) the disease is selected from cancer, immune disease, neurological disease, inflammatory disease, allergy, transplant rejection, viral infection, immune deficiency, and other immune system-related disease cancer.

9. An antibody or fragment thereof for the use of claim 8 wherein the cancer is selected from bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, breast cancer, brain cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, kidney cancer, sarcoma of soft tissue, cancer of the urethra, cancer of the bladder, renal cancer, lung cancer, non-small cell lung cancer, thymoma, prostate cancer, mesothelioma, adrenocortical carcinoma, lymphomas, such as such as Hodgkin's disease, non-Hodgkin's, gastric cancer.

10. An *in vitro* method of reducing tumor cell growth and/or proliferation in a tumor cell or tissue comprising:
a) contacting a tumor cell with an effective amount of an antibody or fragment thereof according to any of claims 1 to 6 or a pharmaceutical composition according to claim 7, wherein the antibody targets an antigen present on the surface of a tumor cell and exerts an inhibitory effect on growth and/or proliferation of the tumor cell when bound to the antigen and/or wherein the antibody that targets an antigen present on the surface of a tumor cell is internalised when bound to the antigen; and
b) irradiating the antibody or fragment with a light source to release the photocaging group and induce specific binding of the antibody to the target antigen; and
thereby inhibiting growth and/or proliferation of the tumor cell.

11. An *in vitro* method of killing a tumor cell comprising:
a) contacting a tumor cell comprising a cell surface protein with a therapeutically effective amount of an antibody or fragment thereof according to claim 1 to 6 or a pharmaceutical composition according to claim 7, wherein the antibody that targets an antigen present on the surface of a tumor cell is internalised when bound to the antigen; and
b) irradiating the antibody or fragment with a light source to release the photocaging group and induce specific binding of the antibody to the target antigen wherein said antibody is internalised upon binding to the antigen; and
thereby killing the cell.

12. The method of claims 10 or 11 wherein the light source is selected from the group consisting of a filtered conventional light source, a diode array and a laser.

13. A method for producing an antibody or antibody fragment according to any of claims to 6, comprising identifying an amino acid residue in one of the CDRs of the antigen binding region for site specific modification and introducing a photocaged group at said position, wherein i) the photocaged amino acid comprises a light-removable protection group; ii) the photocaged amino acid inhibits interaction and/or binding of the antibody to its antigen wherein binding and/or interaction of the antibody to its antigen can be induced upon activation with a light source.

14. A kit comprising the antibody or fragment thereof according to any of claims 1 to 6., optionally comprising a LED wearable device or an LED implantable device.

## Patentansprüche

1. Antikörper oder Fragment davon, wobei der Antikörper oder das Fragment eine Photocaged-Aminosäure in einer der CDRs seiner antigenbindenden Region umfasst, wobei: i) die Photocaged-Aminosäure eine durch Licht entfernbare Schutzgruppe umfasst; ii) die Photocaged-Aminosäure an einer Stelle vorhanden ist, bei der die Photocaged-Aminosäure eine Wechselwirkung des Antikörpers mit seinem Antigen und/oder sein Binden daran hemmt, wobei Binden des Antikörpers an sein Antigen und/oder seine Wechselwirkung damit nach Aktivierung mit einer Lichtquelle induziert werden kann und wobei der Antikörper oder das Fragment davon an eine andere Gruppierung konjugiert ist.

2. Antikörper oder Fragment davon nach Anspruch 1, wobei die Aminosäure eine photoaktive Gruppe enthält, die aus einer o-Nitrobenzyl-funktionelle-Gruppe und Varianten davon ausgewählt ist.

3. Antikörper oder Fragment davon nach Anspruch 1 oder 2, wobei das Fragment ein F(ab')2, Fab, Fv, sFv scFv oder dAbs umfasst.

4. Antikörper oder Fragment davon nach einem der vorhergehenden Ansprüche, wobei die Gruppierung aus einer Halbwertszeit verlängernden Gruppierung, Markierungs- oder toxischen Gruppierung ausgewählt ist, gegebenenfalls
wobei die Gruppierung über eine unnatürliche Aminosäure angebunden ist.

5. Antikörper oder Fragment davon nach einem vorhergehenden Anspruch, wobei der Antikörper oder das Fragment davon an ein Zelloberflächenantigen bindet.

6. Antikörper oder Fragment davon nach einem vorhergehenden Anspruch, wobei der Antikörper oder das Fragment davon an ein tumorassoziiertes Antigen oder ein Immuncheckpoint-Molekül bindet.

7. Pharmazeutische Zusammensetzung, umfassend einen Antikörper oder ein Fragment davon nach einem vorhergehenden Anspruch und gegebenenfalls einen pharmazeutisch unbedenklichen Träger.

8. Antikörper oder Fragment davon nach einem der Ansprüche 1 bis 6 oder pharmazeutische Zusammensetzung nach Anspruch 7 zur Verwendung bei der Krankheitsbehandlung, wobei: i) der Antikörper oder das Fragment davon durch Bestrahlen eines Zielgewebes mit einer Lichtquelle aktiviert werden soll, gegebenenfalls wobei die Wellenlänge 365 nm beträgt; ii) die Krankheit aus Krebs, Immunkrankheit, neurologischer Erkrankung, Entzündungskrankheit, Allergie, Transplantatabstoßung, Virusinfektion, Immunschwäche und anderem Krebs einer mit dem Immunsystem verbundenen Krankheit ausgewählt ist.

9. Antikörper oder Fragment davon zur Verwendung gemäß Anspruch 8, wobei der Krebs aus Knochenkrebs, Bauchspeicheldrüsenkrebs, Hautkrebs, Krebs des Kopf- oder Halsbereichs, kutanem oder intraokularem malignem Melanom, Gebärmutterkrebs, Eierstockkrebs, Rektumkarzinom, Krebs im Analbereich, Magenkrebs, Hodenkrebs, Brustkrebs, Hirnkrebs, Tubenkarzinom, Endometriumkarzinom, Cervixkarzinom, Vaginalkarzinom, Vulvakrebs, Speiseröhrenkrebs, Dünndarmkrebs, Krebs des endokrinen Systems, Schilddrüsenkrebs, Nebenschilddrüsenkrebs, Nebennierenkrebs, Nierentumor, Weichteilsarkom, Harnröhrenkrebs, Blasenkrebs, Nierenkrebs, Lungenkrebs, nichtkleinzelligem Lungenkrebs, Thymom, Prostatakrebs, Mesotheliom, Nebennierenrindenkarzinom, Lymphomen, wie etwa Hodgkin-Krankheit, Non-Hodgkin-Lymphom, Magenkarzinom ausgewählt ist.

10. In-Vitro-Verfahren zum Verringern von Tumorzellwachstum und/oder -proliferation bei einer Tumorzelle oder einem Tumorgewebe, umfassend:
a) In-Kontakt-Bringen einer Tumorzelle mit einer wirksamen Menge eines Antikörpers oder Fragments davon nach einem der Ansprüche 1 bis 6 oder einer pharmazeutischen Zusammensetzung nach Anspruch 7, wobei der Antikörper auf ein auf der Oberfläche einer Tumorzelle vorhandenes Antigen zielt und eine hemmende Wirkung auf Wachstum und/oder Proliferation der Tumorzelle ausübt, wenn er an das Antigen gebunden ist, und/oder wobei der Antikörper, der auf ein auf der Oberfläche einer Tumorzelle vorhandenes Antigen zielt, internalisiert wird, wenn er an das Antigen gebunden ist; und
b) Bestrahlen des Antikörpers oder Fragments mit einer Lichtquelle, sodass die Photocaging-Gruppe freigesetzt und spezifisches Binden des Antikörpers an das Zielantigen induziert wird; und
dadurch Hemmen von Wachstum und/oder Proliferation der Tumorzelle.

11. In-Vitro-Verfahren zum Abtöten einer Tumorzelle, umfassend:
a) In-Kontakt-Bringen einer ein Zelloberflächenprotein umfassenden Tumorzelle mit einer therapeutisch wirksamen Menge eines Antikörpers oder Fragments davon nach Anspruch 1 bis 6 oder einer pharmazeutischen Zusammensetzung nach Anspruch 7, wobei der Antikörper, der auf ein auf der Oberfläche einer Tumorzelle vorhandenes Antigen zielt, internalisiert wird, wenn er an das Antigen gebunden ist; und
b) Bestrahlen des Antikörpers oder Fragments mit einer Lichtquelle, sodass die Photocaging-Gruppe freigesetzt und spezifisches Binden des Antikörpers an das Zielantigen induziert wird, wobei der Antikörper nach Binden an das Antigen internalisiert wird; und
dadurch Abtöten der Zelle.

12. Verfahren gemäß Anspruch 10 oder 11, wobei die Lichtquelle aus der Gruppe bestehend aus einer gefilterten konventionellen Lichtquelle, einem Diodenarray und einem Laser ausgewählt ist.

13. Verfahren zum Herstellen eines Antikörpers oder Antikörperfragments nach einem der Ansprüche 1 bis 6, umfassend Identifizieren eines Aminosäurerestes in einer der CDRs der antigenbindenden Region für stellenspezifische Modifikation und Einführen einer Photocaged-Gruppe an der Position, wobei i) die Photocaged-Aminosäure eine durch Licht entfernbare Schutzgruppe umfasst; ii) die Photocaged-Aminosäure eine Wechselwirkung des Antikörpers mit seinem Antigen und/oder sein Binden daran hemmt, wobei Binden des Antikörpers an sein Antigen und/oder seine Wechselwirkung damit nach Aktivierung mit einer Lichtquelle induziert werden kann.

14. Kit, umfassend den Antikörper oder das Fragment davon nach einem der Ansprüche 1 bis 6, gegebenenfalls umfassend eine tragbare LED-Vorrichtung oder eine implantierbare LED-Vorrichtung.

## Revendications

1. Anticorps ou fragment de celui-ci, dans lequel ledit anticorps ou fragment comprend un acide aminé en photocage dans l'une des CDR de sa région de liaison à un antigène, dans lequel : i) l'acide aminé en photocage comprend un groupe protecteur pouvant être éliminé à la lumière ; ii) l'acide aminé en photocage est présent au niveau d'un site dans lequel l'acide aminé en photocage inhibe l'interaction et/ou la liaison de l'anticorps à son antigène, la liaison et/ou l'interaction de l'anticorps à son antigène pouvant être induites lors de l'activation avec une source lumineuse, et l'anticorps ou son fragment étant conjugué à une autre fraction.

2. Anticorps ou fragment de celui-ci selon la revendication 1, dans lequel ledit acide aminé comprend un groupe photoactif choisi parmi un groupe fonctionnel o-nitrobenzyle et des variantes de celui-ci.

3. Anticorps ou fragment de celui-ci selon la revendication 1 ou 2, dans lequel ledit fragment comprend un F(ab')2, Fab, Fv, sFv scFv ou dAbs.

4. Anticorps ou fragment de celui-ci selon l'une quelconque des revendications précédentes, dans lequel ladite fraction est choisie parmi une fraction d'extension de demi-vie, un marqueur ou une fraction toxique, éventuellement
dans lequel ladite fraction est fixée par l'intermédiaire d'un acide aminé non naturel.

5. Anticorps ou fragment de celui-ci selon une revendication précédente, dans lequel ledit anticorps ou fragment de celui-ci se lie à un antigène de surface cellulaire.

6. Anticorps ou fragment de celui-ci selon une revendication précédente, dans lequel ledit anticorps ou fragment de celui-ci se lie à un antigène associé à une tumeur ou à une molécule de point de contrôle immunitaire.

7. Composition pharmaceutique comprenant un anticorps ou un fragment de celui-ci selon une revendication précédente et éventuellement un support pharmaceutiquement acceptable.

8. Anticorps ou fragment de celui-ci selon l'une quelconque des revendications 1 à 6 ou composition pharmaceutique selon la revendication 7 pour une utilisation dans le traitement d'une maladie, dans lequel : i) l'anticorps ou le fragment de celui-ci doit être activé par irradiation d'un tissu cible avec une source lumineuse, éventuellement dans lequel la longueur d'onde est de 365 nm ; ii) la maladie est choisie parmi le cancer, une maladie immunitaire, une maladie neurologique, une maladie inflammatoire, une allergie, un rejet de greffe, une infection virale, un déficit immunitaire et une autre maladie cancéreuse liée au système immunitaire.

9. Anticorps ou fragment de celui-ci pour l'utilisation selon la revendication 8, dans lequel le cancer est choisi parmi le cancer des os, le cancer du pancréas, le cancer de la peau, le cancer de la tête ou du cou, le mélanome malin cutané ou intraoculaire, le cancer de l'utérus, le cancer de l'ovaire, le cancer du rectum, le cancer de la région anale, le cancer de l'estomac, le cancer des testicules, le cancer du sein, le cancer du cerveau, le carcinome des trompes de Fallope, le carcinome de l'endomètre, le carcinome du col de l'utérus, le carcinome du vagin, le carcinome de la vulve, le cancer de l'oesophage, le cancer de l'intestin grêle, le cancer du système endocrinien, le cancer de la glande thyroïde, le cancer de la glande parathyroïde, le cancer de la glande surrénale, le cancer du rein, le sarcome des tissus mous, le cancer de l'urètre, le cancer de la vessie, le cancer du rein, le cancer du poumon, le cancer du poumon non à petites cellules, le thymome, le cancer de la prostate, le mésothéliome, le carcinome corticosurrénal, les lymphomes tels que la maladie de Hodgkin, la maladie non hodgkinienne, le cancer gastrique.

10. Procédé *in vitro* de réduction de la croissance et/ou de la prolifération de cellules tumorales dans une cellule ou un tissu tumoral comprenant :
a) la mise en contact d'une cellule tumorale avec une quantité efficace d'un anticorps ou d'un fragment de celui-ci selon l'une quelconque des revendications 1 à 6 ou d'une composition pharmaceutique selon la revendication 7, dans lequel l'anticorps cible un antigène présent sur la surface d'une cellule tumorale et exerce un effet inhibiteur sur la croissance et/ou la prolifération de la cellule tumorale lorsqu'il est lié à l'antigène et/ou dans lequel l'anticorps qui cible un antigène présent sur la surface d'une cellule tumorale est internalisé lorsqu'il est lié à l'antigène ; et
b) l'irradiation de l'anticorps ou du fragment avec une source lumineuse pour libérer le groupe de mise en photocage et induire une liaison spécifique de l'anticorps à l'antigène cible ; et
inhibant ainsi la croissance et/ou la prolifération de la cellule tumorale.

11. Procédé *in vitro* de destruction d'une cellule tumorale comprenant :
a) la mise en contact d'une cellule tumorale comprenant une protéine de surface cellulaire avec une quantité thérapeutiquement efficace d'un anticorps ou d'un fragment de celui-ci selon les revendications 1 à 6 ou d'une composition pharmaceutique selon la revendication 7, dans lequel l'anticorps qui cible un antigène présent sur la surface d'une cellule tumorale est internalisé lorsqu'il est lié à l'antigène ; et
b) l'irradiation de l'anticorps ou du fragment avec une source lumineuse pour libérer le groupe de mise en photocage et induire une liaison spécifique de l'anticorps à l'antigène cible, ledit anticorps étant internalisé lors de la liaison à l'antigène ; et
détruisant ainsi de la cellule.

12. Procédé selon la revendication 10 ou 11, dans lequel la source lumineuse est choisie dans le groupe constitué par une source lumineuse conventionnelle filtrée, un réseau de diodes et un laser.

13. Procédé de production d'un anticorps ou d'un fragment d'anticorps selon l'une quelconque des revendications 1 à 6, comprenant l'identification d'un résidu d'acide aminé dans l'une des CDR de la région de liaison à un antigène pour une modification spécifique d'un site et l'introduction d'un groupe en photocage au niveau de ladite position, dans lequel i) l'acide aminé en photocage comprend un groupe protecteur pouvant être éliminé à la lumière ; ii) l'acide aminé en photocage inhibe l'interaction et/ou la liaison de l'anticorps à son antigène, la liaison et/ou l'interaction de l'anticorps à son antigène pouvant être induites lors de l'activation avec une source lumineuse.

14. Kit comprenant l'anticorps ou le fragment de celui-ci selon l'une quelconque des revendications 1 à 6, comprenant éventuellement un dispositif portable à LED ou un dispositif implantable à LED.
